# EUROPEAN PATENT APPLICATION

(11) **EP 4 643 791 A1**
(43) Date of publication of application: **05.11.2025**
(21) Application number: 25173498.4
(22) Date of filing: 30.04.2025
(51) Int. Cl.: A61B 17/04, A61B 17/34

(54) **ANCHOR AND ANCHORING DEVICE**

(30) Priority: 30.04.2024 CN 202410545893
(71) Applicant: Micro-Tech (Nanjing) Co., Ltd., Nanjing, Jiangsu 210032 (CN)
(72) Inventor: JIN, Hongyan, NANJING, 210032 (CN); WEI, Jianyu, NANJING, 210032 (CN); MA, Xiaojun, NANJING, 210032 (CN); CHENG, Xue, NANJING, 210032 (CN); HU, Jie, NANJING, 210032 (CN)
(74) Representative: De Giorgi, Michele

(57) **Abstract**

Provided are an anchor and an anchoring device, including: a traction line, which is a flexible member; an anchoring body connected to a distal end of the traction line; a driving tube, which is at least sleeved on at least a portion of the traction line. The anchoring body is configured to move along a puncture needle under the drive of the driving tube, and can be flipped when extending out of the distal end of the puncture needle to form an angle with the traction line, so that the anchoring body stops at the inner wall of the target tissue. The anchor and the anchoring device can smoothly implant the anchoring body of the anchor into the target tissue.

## Description

### TECHNICAL FIELD

The present invention relates to the technical field of medical device, and in particular to an anchor and an anchoring device.

### BACKGROUND ART

Endoscopic Ultrsonography (EUS for short) is a minimally invasive surgery for evaluating digestive tract and lung diseases. It involves medical surgeries on the patient's gastrointestinal tract. For example, in gallbladder drainage surgery guided by endoscopic ultrsonography, a fixator needs to be implanted between the gallbladder and the intestine. The fixator is used as a fistula to achieve communication between the gallbladder and the intestine.

In order to quickly install the fixator, an anchor is usually implanted into the gallbladder through the intestine under the guidance of an endoscope. By pulling the traction line of the anchor outward, the anchoring body of the anchor brings the gallbladder to tightly adhere to the side wall of the intestine. How to more smoothly implant the anchoring body into the target tissue, such as the gallbladder, is a problem that needs to be solved urgently.

### SUMMARY

The embodiment of the present invention provides an anchor and an anchoring device, which can smoothly implant the anchoring body of the anchor into the target tissue.

On the one hand, the embodiment of the present invention provides an anchor, including:
a traction line, which is a flexible member;
an anchoring body, connected to the distal end of the traction line, and capable of being pulled by the traction line; and
a driving tube, at least sleeved on at least a portion of the traction line, wherein
the anchoring body is configured to move along a puncture needle under the drive of the driving tube, and can be flipped when extending out of the distal end of the puncture needle to form an angle with the traction line, so that the anchoring body stops at the inner wall of the target tissue.

In one embodiment, the driving tube is sleeved on the traction line, and the anchoring body is located on the distal end of the driving tube; and
when the anchoring body is located in the puncture needle, the proximal end of the anchoring body abuts the distal end of the driving tube, so that when the anchoring body extends out of the puncture needle, the anchoring body can be flipped under the pushing of the driving tube and the pulling of the traction line.

In one embodiment, the anchor further includes a distal locking structure, which is configured to be fixed to the proximal end of the puncture needle, a first receiving channel is formed in the distal locking structure, and the first receiving channel communicates with a needle channel of the puncture needle, wherein a part of the driving tube and the anchoring body are located in the first receiving channel, and the anchoring body is configured to enter the needle channel of the puncture needle through the first receiving channel under the drive of the driving tube; and
the distal locking structure is configured to selectively cooperate with the driving tube to lock the driving tube and the anchoring body in the first receiving channel.

In one embodiment, the distal locking structure includes:
a distal connecting member, configured to be fixed to the proximal end of the puncture needle and to form at least a portion of the first receiving channel for the driving tube and the anchoring body to pass through; and
a distal locking member, disposed on the distal connecting member and configured to selectively cooperate with the driving tube to lock or release the driving tube and the anchoring body on the distal connecting member.

In one embodiment, the distal locking member includes:
a distal fixing portion, which is provided with a first receiving channel and is selectively fixed to or released from the distal connecting member; and
a distal locking portion, which is located in the distal fixing portion, wherein
when the distal fixing portion is fixed to the distal connecting member, the distal locking portion is configured to cooperate with the distal connecting member and act on the driving tube to lock the driving tube and the anchoring body; and
when the distal fixing portion is released from the distal connecting member, the distal locking portion is configured to detach from the distal connecting member to release the driving tube and the anchoring body.

In one embodiment, the distal locking portion is a distal elastic member embedded in the distal fixing portion, and the distal elastic member is elastic at least along a first direction; the first direction intersects with an extension direction of the driving tube;
when the distal fixing portion is fixed to the distal connecting member, the distal elastic member is pressed by the distal connecting member and abuts the driving tube along the first direction to lock the driving tube and the anchoring body; and
when the distal fixing portion is released from the distal connecting member, the distal elastic member detaches from the distal connecting member to release the driving tube and the anchoring body during the rebound process.

In one embodiment, the distal elastic member is a distal elastic sleeve;
the distal elastic sleeve is provided with a first penetration hole for the driving tube to pass through; and
under the pressing by the distal connecting member, a hole wall of the first penetration hole of the distal elastic sleeve abuts the driving tube to lock the driving tube and the anchoring body, and after the distal elastic sleeve is detached from the distal connecting member, the hole wall of the first penetration hole rebounds to release the driving tube and the anchoring body.

In one embodiment, the distal fixing portion is a distal locking nut, and the distal locking nut is threadedly connected to the distal connecting member.

In one embodiment, the anchor further includes:
a proximal locking structure, which is arranged at the proximal end of the driving tube and fixedly connected to the driving tube, wherein
a second receiving channel is formed in the proximal locking structure, and the traction line is passed through the second receiving channel; and
the proximal locking structure is configured to selectively cooperate with the traction line to lock the traction line on the driving tube.

In one embodiment, the proximal locking structure includes:
a proximal connecting member, fixed to the proximal end of the driving tube and forming at least a portion of the second receiving channel for the traction line to pass through; and
a proximal locking member, which is arranged on the proximal connecting member and configured to selectively cooperate with the traction line to lock or release the traction line on the proximal connecting member.

In one embodiment, the proximal locking member includes:
a proximal fixing portion, which is provided with a second receiving channel and selectively fixed to or released from the proximal connecting member; and
a proximal locking portion, which is located in the proximal fixing portion, wherein
when the proximal fixing portion is fixed to the proximal connecting member, the proximal locking portion is configured to cooperate with the proximal connecting member and act on the traction line to lock the traction line; and
when the proximal fixing portion is released from the proximal connecting member, the proximal locking portion is configured to detach from the proximal connecting member to release the traction line.

In one embodiment, the proximal locking portion is a proximal elastic member embedded in the proximal fixing portion, and the proximal elastic member is elastic at least along the first direction; the first direction intersects with the extension direction of the traction line;
when the proximal fixing portion is fixed to the proximal connecting member, the proximal elastic member is pressed by the proximal connecting member and abuts the traction line along the first direction to lock the traction line; and
when the proximal fixing portion is released from the proximal connecting member, the proximal elastic member is detached from the proximal connecting member to release the traction line during the rebound process.

In one embodiment, the proximal elastic member is a proximal elastic sleeve;
the proximal elastic sleeve is provided with a second penetration hole for the traction line to pass through; and
when the proximal elastic sleeve is pressed by the proximal connecting member, a hole wall of the second penetration hole abuts the traction line to lock the driving tube, and after the proximal elastic sleeve is detached from the proximal connecting member, the hole wall of the penetration hole rebounds to release the traction line.

In one embodiment, the proximal fixing portion is a proximal locking nut, and the proximal locking nut is threadedly connected to the proximal connecting member.

In one embodiment, the anchor further includes:
a stopper, selectively arranged between the driving tube and the puncture needle, wherein
when the anchoring body is located in the puncture needle and has a preset distance from the distal end of the puncture needle, the stopper cooperates with the driving tube and the puncture needle respectively to limit the movement of the driving tube in the puncture needle; and
during the movement of the anchoring body from a first position to the distal end of the puncture needle, the stopper is detached from the position between the driving tube and the puncture needle, wherein the first position is the position of the anchoring body when it has a preset distance from the distal end of the puncture needle.

In one embodiment, the stopper is arranged between the proximal locking structure and the distal locking structure of the anchor and selectively protrudes from the side wall of the driving tube;
at least when the anchoring body is located in the puncture needle and has a preset distance from the distal end of the puncture needle, the stopper protrudes from the side wall of the driving tube so that the proximal end of the driving tube is blocked by the stopper on the proximal end of the puncture needle; and
during the movement of the anchoring body from the first position to the distal end of the puncture needle, the stopper is detached from the side wall of the driving tube.

In one embodiment, the stopper is detachably disposed on the driving tube; and
when the anchoring body moves from the first position toward the distal end of the puncture needle, the stopper is configured to be detached from the driving tube.

In one embodiment, the stopper is disposed at one end of the proximal locking structure of the anchor toward the distal locking structure; and
the preset distance is 0, and the length of the stopper is equal to the maximum length of the anchoring body extending out of the distal end of the puncture needle.

In one embodiment, a limiting groove and a side notch communicated with the limiting groove are formed on the anchoring body, the limiting groove and the side notch both extend along the length direction of the anchoring body, and one ends of the limiting groove and the side notch both run through the proximal end of the anchoring body; and
when the proximal end of the anchoring body is located at the puncture needle, at least part of the traction line is located in the limiting groove, and during the process of the traction line pulling the anchoring body to flip, a part of the traction line detaches from the anchoring body through the side notch.

In one embodiment, an avoidance opening is formed at one end of the limiting groove located at the proximal end of the anchoring body, one end of the avoidance opening extends to the side notch, and the other end of the avoidance opening extends to the side of an axis of the limiting groove away from the side notch.

In one embodiment, there is a gap between the other end of the avoidance opening and the groove bottom of the limiting groove,
wherein the groove bottom of the limiting groove is arranged opposite to the side notch.

In one embodiment, when the proximal end of the anchoring body is located at the puncture needle, the traction line extends along the axis of the limiting groove; or
when the proximal end of the anchoring body is located at the puncture needle, the traction line is located between the axis of the limiting groove and the side notch.

In one embodiment, a line placement cavity is formed on the anchoring body; and
the line placement cavity communicates with the groove cavity of the limiting groove through a penetration hole, the distal end of the traction line is limited in the line placement cavity, and the traction line extends from the penetration hole into the limiting groove.

In one embodiment, a first limiting portion is formed at the distal end of the traction line, and the first limiting portion is embedded in the line placement cavity so that the distal end of the traction line is limited in the line placement cavity.

In one embodiment, a side wall of the anchoring body is recessed inwardly to form a line placement groove, and a groove cavity of the line placement groove is configured as the line placement cavity.

In one embodiment, a mounting channel is formed in the anchoring body, one end of the mounting channel communicates with the penetration hole, and the other end of the mounting channel penetrates to the distal end of the anchoring body;
the distal end of the anchoring body has an end cap, the end cap is embedded in the mounting channel from the distal end of the anchoring body, and one end is spaced from the penetration hole; and
the line placement cavity is formed between one end of the end cap and the penetration hole.

On the other hand, the embodiment of the present invention also provides an anchoring device, including:
a puncture needle, the distal end of which is configured to extend into a target tissue; and
an anchor as above-mentioned, wherein
the anchoring body of the anchor moves along the puncture needle under the drive of the driving tube to enter the target tissue.

The embodiment of the present invention provides an anchor and an anchoring device, the traction line is arranged as a flexible member, and the traction line is disposed in a driving tube, so that when the anchoring body at one end of the traction line needs to be implanted, the puncture needle can be implanted into the target tissue first, and then the traction line and the anchoring body can be driven by the driving tube to move along the puncture needle toward the target tissue. When the anchoring body extends out of the distal end of the puncture needle, the anchoring body can be flipped to have an angle with the traction line, so that the anchoring body can stop at the inner wall of the target tissue, so that the traction line can be pulled so that the target tissue is closely attached to other tissues under the drive of the anchoring body. The providing of the driving tube can make the flexible traction line and the anchoring body be smoothly implanted into the target tissue through the puncture needle.

### BRIEF DESCRIPTION OF DRAWINGS

Figure 1 is a schematic diagram of the structure of one of the anchoring devices provided in one embodiment of the present invention;
Figure 2a is a schematic diagram of the structure of another anchoring device provided in one embodiment of the present invention;
Figure 2b is a partial enlarged view of part A in Figure 2a;
Figure 3 is a schematic diagram of the overall structure of the anchor provided in one embodiment of the present invention;
Figure 4 is a sectional view of the distal locking structure in Figure 3;
Figure 5 is a partial schematic diagram of part B in Figure 4;
Figure 6 is a schematic diagram of the structure of the anchor provided in one embodiment of the present invention in a first state;
Figure 7 is a schematic diagram of the structure of the anchor provided in one embodiment of the present invention in a second state;
Figure 8 is a sectional view of the proximal locking structure in Figure 3;
Figure 9 is a schematic diagram of the coordination of the proximal locking structure and a stopper in Figure 3;
Figure 10 is a schematic diagram of the structure of one of the anchoring bodies provided in one embodiment of the present invention;
Figure 11 is a sectional view of the assembly of the anchoring body and the traction line in Figure 10;
Figure 12 is a sectional view of the anchoring body in Figure 11;
Figure 13 is a partial enlarged view of part C in Figure 6;
Figure 14 is a schematic diagram of the structure of another anchoring body provided in one embodiment of the present invention.

Explanation of reference numerals:
10-anchor; 20-puncture needle;
100-traction line; 200-anchoring body; 300-driving tube; 400-distal locking structure; 500-proximal locking structure; 600-stopper; 21-fixing portion;
410-distal connecting member; 420-distal locking member; 430-first receiving channel; 510-proximal connecting member; 520-proximal locking member; 530-second receiving channel;
411-guide portion; 421-distal fixing portion; 422-distal locking portion; 423-first through hole; 521-proximal fixing portion; 522-proximal locking portion; 523-second through hole;
110-first limiting portion; 210-limiting groove; 220-line placement cavity; 220a-line placement groove; 240-avoidance opening; 250-end cap; 260-mounting channel; 410-second limiting portion;
210a-groove bottom; 210b-side notch; P-flip fulcrum; 210c-penetration hole.

### DETAILED DESCRIPTION OF EMBODIMENTS

In order to enable those skilled in the art to better understand the technical solutions in the present invention, the technical solutions in the embodiments of the present invention will be clearly and completely described below in conjunction with the drawings in the embodiments of the present invention. Obviously, the described embodiments are only part of the embodiments of the present invention, not all of the embodiments. Based on the embodiments in the present invention, all other embodiments obtained by those ordinary skilled in the art without creative work should fall within the scope of protection of the present invention.

It should be noted that many specific details are described in the following description to facilitate a full understanding of the present invention. However, the present invention can also be implemented in other ways different from those described herein. Therefore, the scope of protection of the present invention is not limited by the specific implementations disclosed below.

In the description of the present invention, it should be understood that the terms "above", "below", "horizontal", "bottom", "inner", "outer" and the like indicate the orientation or position relationship based on the orientation or position relationship shown in the drawings, which is only for the convenience of describing the present invention and simplifying the description, rather than indicating or implying that the device or element referred to must have a specific orientation, be constructed and operated in a specific orientation, and therefore cannot be understood as a limitation on the present invention. In this application, unless otherwise clearly specified and limited, the first feature "above" or "below" the second feature may be the first and second features in direct contact, or the first and second features in indirect contact through an intermediate medium.

In this application, unless otherwise clearly specified and limited, the terms "connected with", "connected to", "fixed to" and the like should be understood in a broad sense, for example, it can be a fixed connection, a detachable connection, or an integral body; it can be directly connected, or indirectly connected through an intermediate medium, it can be the internal connection of the two elements or the interaction relationship between the two elements. However, the direct connection indicates that the two connected bodies do not establish a connection relationship through a transition structure, but are only connected to form a whole through a connecting structure. For those ordinary skilled in the art, the specific meanings of the above terms in this application can be understood according to the specific circumstances.

In this application, the descriptions involving "first", "second", etc. are only for descriptive purposes, and cannot be understood as indicating or implying their relative importance or implicitly indicating the number of technical features indicated. Therefore, the features defined as "first" and "second" can explicitly or implicitly include at least one of the features.

Endoscopic Ultrsonography (EUS) is a minimally invasive surgery for evaluating digestive tract and lung diseases. In medical surgeries involving the gastrointestinal tract of patients, it is necessary to implant a fixator between the two tissues for drainage, and use the fixator as a fistula to achieve the communication between the two tissues. For example, in gallbladder drainage surgery guided by endoscopic ultrsonography, it is necessary to implant a fixator between the gallbladder and the intestine, and use the fixator as a fistula to achieve the communication between the gallbladder and the intestine.

It can be understood that the gallbladder is in a free state, which makes it inconvenient to quickly install the fixator between the gallbladder and the intestine.

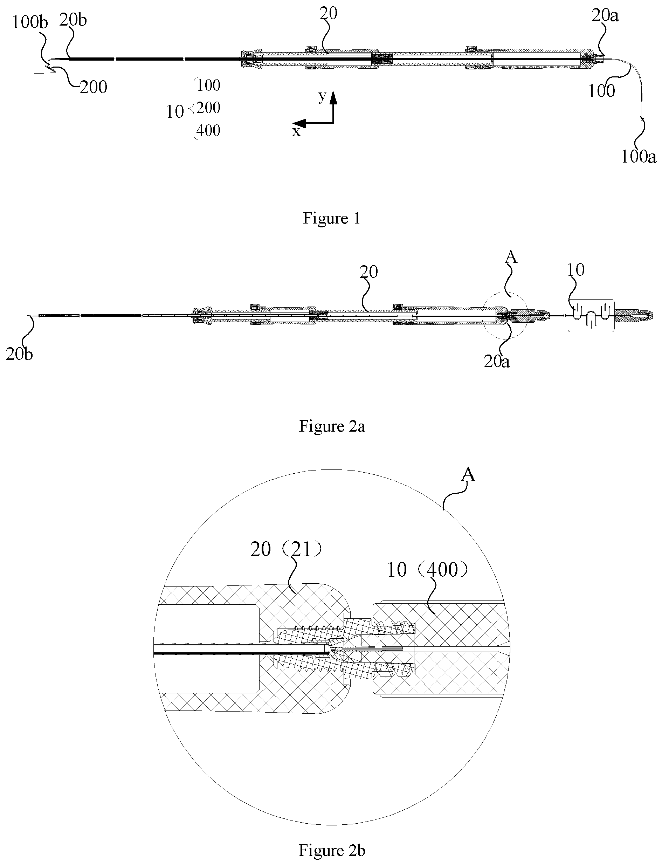

In order to ensure the rapid installation of the fixator, an embodiment of the present invention provides an endoscopic ultrasonic anchoring system, including an endoscope and an anchoring device. Figure 1 is a schematic diagram of the structure of one of the anchoring devices provided in an embodiment of the present invention. As shown in Figure 1, the anchoring device includes an anchor 10, and the anchor 10 has a traction line 100 and an anchoring body 200. The anchoring body 200 is connected to the distal end (refer to 100b in Figure 1) of the traction line 100.

When a surgery is required, firstly, the anchoring body 200 can be implanted into the target tissue such as the gallbladder through the first tissue such as the intestine under the guidance of the endoscope. For example, the cannula of the endoscope can be introduced into the intestine first, and then the anchor 10 can be sent along instrument channel of the cannula. When the anchoring body 200 of the anchor 10 reaches the gallbladder through the intestine, the traction line 100 is pulled outward so that the anchoring body 200 stops on the inner wall of the target tissue, and the traction line 100 is continued to be pulled so that the anchoring body 200 brings the gallbladder to be tightly attached to the side wall of the intestine, that is, the gallbladder is anchored, so that the gallbladder is firmly attached to the outside of the intestine. On the one hand, it is convenient to quickly install the fixator between the intestine and the gallbladder, and on the other hand, it also makes the fixator more stable between the intestine and the gallbladder.

In some examples, in order to achieve the implantation of the anchor 10, the anchor 10 may include an outer tube and an inner tube, wherein the inner tube is placed inside the outer tube, the traction line 100 of the anchor 10 is located in the inner tube, the anchoring body 200 of the anchor 10 is located in the outer tube, and the proximal end of the anchoring body 200 abuts against the distal end of the inner tube. When the anchoring body 200 needs to be implanted, the inner tube, the anchoring body 200 and the traction line 100 inside the outer tube and the inner tube can be implanted into the intestine along the cannula of the endoscope through the outer tube, and then the outer tube is kept stationary, and the inner tube is pushed so that the anchoring body 200 on the distal end of the inner tube gradually extends out of the outer tube, passes through the intestine and the side wall of the gallbladder, until it reaches the gallbladder, and then the traction line 100 is pulled so that the anchoring body 200 stops at the side wall of the gallbladder, and the traction line 100 is continued to be pulled so that the gallbladder is to stick close to the intestine.

It can be understood that, in the above example, the anchoring body 200 not only plays the role of pulling the gallbladder, but also serves the purpose of puncturing the tissue. During the process of anchoring body 200 puncturing tissue, it is difficult for the endoscope to develop anchoring body 200, and therefore the position of anchoring body 200 cannot be determined, which in turn affects the implantation efficiency of anchoring body 200. In addition, the implantation distance of anchoring body 200 may be not enough. For example, when anchoring body 200 is implanted on the side wall of the gallbladder but not fully extended into the gallbladder, while the traction line 100 is pulled, anchoring body 200 will flip, but the gallbladder will be damaged during the flipping process. In addition, the implantation distance of anchoring body 200 may be too large, thereby damaging tissues such as the inner wall of the gallbladder.

In addition, in the above example, the distal end of the traction line 100 is fixed to the middle position of the anchoring body 200 to ensure that the anchoring body 200 can achieve better flipping during the pulling process of the traction line 100. In order to enable the traction line 100 to be introduced into the inner tube from the middle position of the anchoring body 200, a notch can be provided on the anchoring body 200, and the notch runs from the middle position to the proximal end of the anchoring body 200, so that the traction line 100 is introduced into the inner tube through the notch.

However, the above-mentioned notch will cause the abutment position between the inner tube and the proximal end of the anchoring body 200 to deviate from the movement axis of the inner tube, which makes it very easy for the inner tube to push the anchoring body 200 to deviate from the movement axis of the inner tube when pushing the anchoring body 200 to puncture the tissue. In other words, the anchoring body 200 is very likely to have an angle with the inner tube during the pushing process of the inner tube, so that the anchoring body 200 cannot smoothly enter the gallbladder.

Figure 2a is a schematic diagram of the structure of another anchoring device provided in an embodiment of the present invention, and Figure 2b is a partial enlarged view of part A in Figure 2a. As shown in Figure 1, Figure 2a and Figure 2b, in some other examples, the anchoring device may further include a puncture needle 20. When the anchor 10 needs to be implanted, the puncture needle 20 may be implanted sequentially through the intestine and gallbladder under the guidance of an endoscope. When the distal end of the puncture needle 20, i.e., the needle tip, reaches the preset position in the gallbladder, the anchor 10 is moved along the needle channel of the puncture needle 20 until the anchoring body 200 of the anchor 10 extends out of the distal end of the puncture needle 20, thereby achieving the purpose of implanting the anchoring body 200 into the gallbladder.

In order to facilitate the implantation of the anchor 10, in some examples, the traction line 100 is formed of a hard material, and there is a resilience force between the traction line 100 and the anchoring body 200, i.e., the anchoring body 200 has a force to resile toward the anchoring position, wherein the anchoring position is the position when the anchoring body 200 has a preset angle with the traction line 100. During the implantation process, the anchoring body 200 moves along the needle channel of the puncture needle 20 under the push of the hard traction line 100 until the anchoring body 200 extends out of the needle head of the puncture needle 20. The anchoring body 200 will have a preset angle with the traction line 100 under the action of the resilience force, thereby pulling the traction line 100, so that the anchoring body 200 can bring the gallbladder to stick close to the intestine.

However, in the above example, during the movement of the anchor 10 along the puncture needle 20, the anchoring body 200 will be parallel to the traction line 100 or have a storage angle less than the preset angle under the constraint of the puncture needle 20. However, because the anchoring body 200 has a resilience force to recover towards the anchoring position, the acting force between the anchoring body 200 and the inner wall of the needle channel of the puncture needle 20 is large, so that the anchoring body 200 has a large resistance with the inner wall of the needle channel of the puncture needle 20 during the movement of anchoring body 200, thereby affecting the smooth implantation of the anchor 10.

In addition, because the traction line 100 is made of hard material, it needs to push the anchoring body 200 to move along the puncture needle 20. Therefore, the diameter of the traction line 100 is relatively large. For example, in this example, the diameter of the hard traction line 100 can be 0.63 mm. In order to avoid occupying too much space in the instrument channel of the endoscope, in the actual operation, after the anchoring body 200 is implanted into the target tissue and before the fixator is implanted, the endoscope is first withdrawn and then inserted to the side of the traction line, so that the traction line is located outside the instrument channel of the endoscope, and then the fixator is implanted through the instrument channel of the endoscope. The entire operation process is cumbersome and complicated.

In addition, when the re-implanting of the endoscope is not needed, the size of the fixator needs to be reduced, which affects the installation of the fixator and the actual drainage effect.

The embodiment of the present invention provides an anchoring device and an anchor. As the anchor at one end of the traction line needs to be implanted, by setting the traction line as a flexible member and setting the traction line in a driving tube, the puncture needle can be implanted into the target tissue first, and then the traction line and the anchoring body can be driven by the driving tube to move along the puncture needle toward the target tissue until the anchoring body extends out of the distal end of the puncture needle. The anchoring body can be flipped under the pull of the traction line to form an angle with the traction line, so that the anchoring body can stop at the inner wall of the target tissue, thereby the traction line can be pulled continuously, so that the target tissue can be closely attached to other tissues under the drive of the anchoring body. The setting of the driving tube can make the flexible traction line and the anchoring body be smoothly implanted into the target tissue through the puncture needle.

In addition, by setting the traction line as a flexible member, the resilience force between the flexible member and the anchoring body is avoided, so that the anchoring body will not excessively abut against the side wall of the puncture needle when moving in the puncture needle, reducing the friction between the anchoring body and the puncture needle, ensuring that the anchoring body moves stably along the puncture needle under the drive of the driving tube, thereby improving the implantation stability and implantation efficiency of the anchoring body. In addition, because the puncture needle is implanted in advance, the anchoring body of the embodiment of the present invention does not need to puncture the tissue, but only needs to move along the puncture needle to the inside of the gallbladder, thereby avoiding the case of the anchoring body implanted in the target tissue at an angle, further improving the implantation efficiency and implantation success rate of the anchoring body.

The following is a detailed description of the structure of the anchoring device and the anchor provided in the embodiment of the present invention in conjunction with the accompanying drawings.

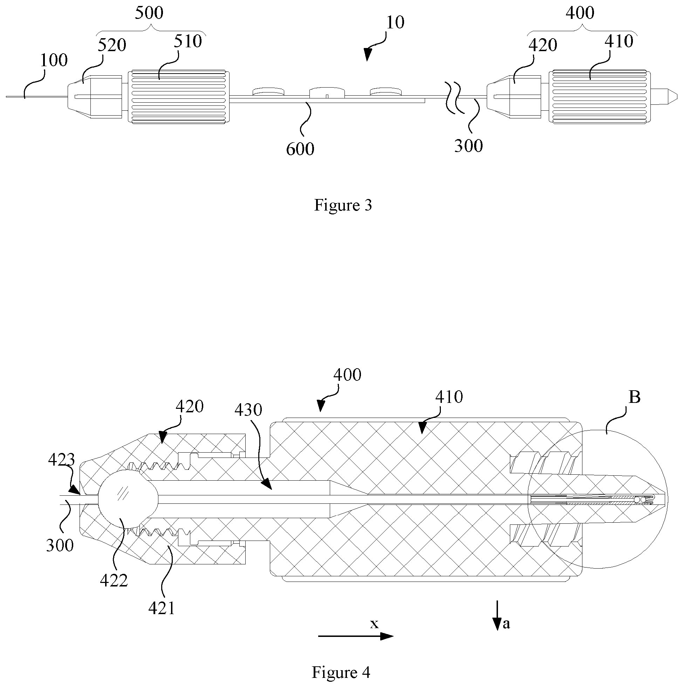

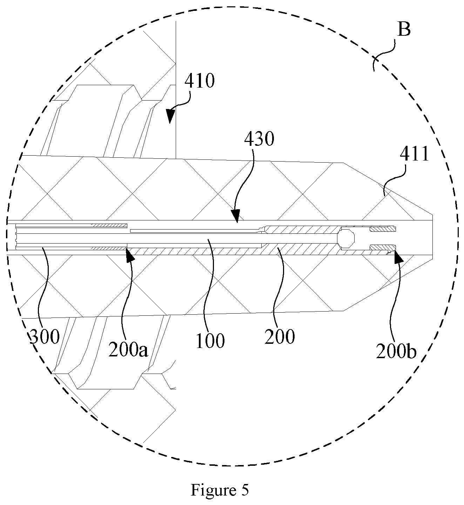

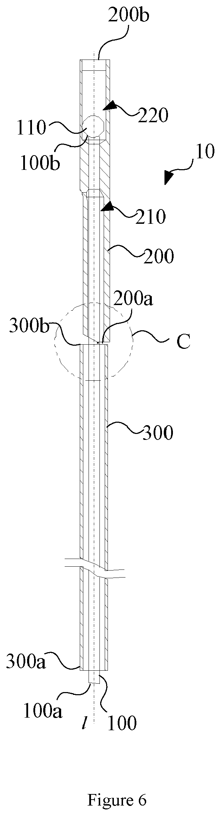

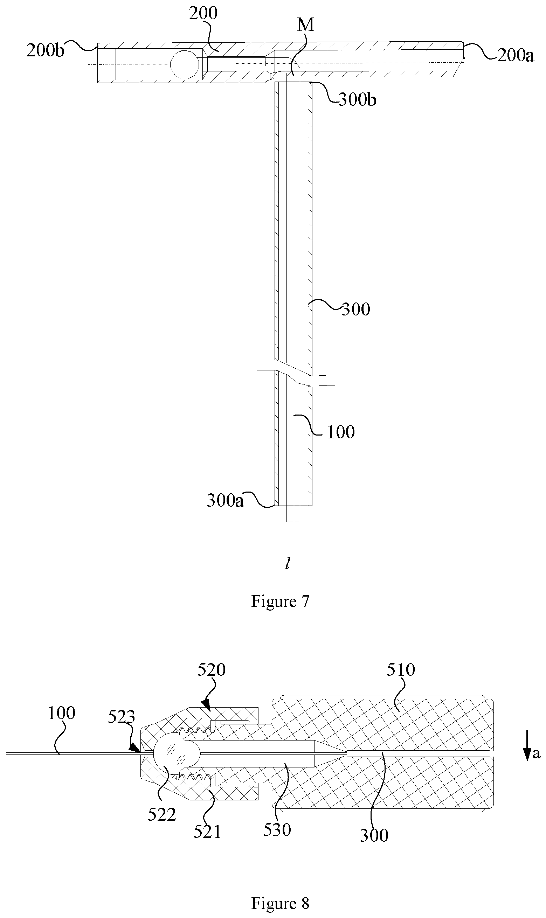

Figure 3 is a schematic diagram of the overall structure of the anchor provided in an embodiment of the present invention, Figure 4 is a sectional view of the distal end of locking structure in Figure 3, Figure 5 is a partial schematic diagram of part B in Figure 4, Figure 6 is a schematic diagram of the structure of the anchor provided in an embodiment of the present invention in the first state, and Figure 7 is a schematic diagram of the structure of the anchor provided in an embodiment of the present invention in the second state. As shown in Figures 1 to 7, an embodiment of the present invention provides an anchoring device, including an anchor 10 and a puncture needle 20. The distal end of the puncture needle 20 (as shown with 20b in Figure 1) is configured to extend into inside the target tissue. For example, when the anchor 10 needs to be implanted, the puncture needle 20 can be first implanted into a first tissue such as the intestine along the cannula of the endoscope, and then the puncture needle 20 can be implanted, so that the distal end of the puncture needle 20 passes through the tissue arm of the first tissue and the tissue wall of the target tissue, and finally reaches a preset position in the target tissue such as the gallbladder. The preset position is a position where the distal end of the puncture needle 20 can be better developed.

As an example, the puncture needle 20 may include but is not limited to a 19G ultrasonic needle.

In the embodiment of the present invention, the anchor 10 includes a traction line 100, which is a flexible member. For example, the traction line 100 can be a flexible line made of fiber material or other flexible materials. The embodiment of the present invention does not limit the material of the traction line 100, and only needs to ensure that the traction line 100 is a flexible member.

The anchor 10 includes an anchoring body 200, which is connected to the distal end of the traction line 100 and can be pulled by the traction line 100.

In some examples, the anchoring body 200 can be flipped under the pull of the traction line 100, so that there is an anchoring angle between the anchoring body 200 and the traction line 100.

In other examples, the anchoring body 200 can also be flipped in other ways, for example, the anchoring body 200 can be flipped by a driving member arranged at the distal end of the puncture needle 20 or the traction line 100. The embodiment of the present invention does not limit the flipping way of the anchoring body 200.

The anchoring angle is the angle between the anchoring body 200 and the traction line 100 when the target tissue, such as the gallbladder, is pulled close to the intestine. As an example, the anchoring angle can be 90° or 90°±10°, which can be adjusted according to the angle of the side wall of the gallbladder, so that the anchoring body 200 fits the side wall of the gallbladder. The embodiment of the present invention does not limit the anchoring angle.

As shown in Figures 3 and 4, the anchor 10 of the embodiment of the present invention includes a driving tube 300, which is at least sleeved on at least a portion of the traction line 100. The anchoring body 200 can move along the puncture needle 20 under the drive of the driving tube 300. For example, the anchoring body 200 can enter the needle channel of the puncture needle 20 from the proximal end of the puncture needle 20 (refer to 20a in Figure 1) driven by the driving tube 300, and move along the needle channel of the puncture needle 20. When extending out of the distal end of the puncture needle 20, the anchoring body 200 can be flipped under the pull of the traction line 100 to have an angle such as an anchoring angle with the traction line 100, so that the anchoring body 200 stops at the inner wall of the target tissue, thereby the traction line 100 is pulled continually, so that the target tissue is closely attached to the outer wall of the first tissue under the drive of the anchoring body 200, and the anchoring of the target tissue is completed.

For the convenience of description, the extension direction of the needle channel of the puncture needle 20 can be the x direction, and the radial direction of the needle channel can be the y direction. The radial dimension of the driving tube 300 can be less than or equal to the radial dimension of the puncture needle 20 along the y direction, so that the driving tube 300 can move smoothly along the needle channel of the puncture needle 20.

In some examples, the driving tube 300 can be sleeved on the first part of the traction line 100. When the anchoring body 200 is located outside the driving tube 300, the first part can be the part between the distal end 100b and the proximal end 100a of the traction line 100, so that a part of the traction line 100 close to the proximal end can be exposed on the proximal end of the driving tube 300, so that the operator can pull the traction line 100 from the proximal end of the traction line 100, thereby the traction line 100 drives the anchoring body 200 to flip, and the part of the traction line 100 close to the distal end also is located outside the driving tube 300 and can be connected to the anchoring body 200 to achieve the flipping of the anchoring body 200.

When the anchoring body 200 is located inside the driving tube 300, the first part can also be the part between the distal end of the traction line 100 and the position of the first length from the distal end. As an example, the first length can be a suitable length such as 2/3 or 3/4 of the total length of the traction line 100. That is to say, the proximal end of the traction line 100 (as shown in 100a in Figure 1) and the position of the second length from the proximal end can be exposed on the proximal end of the driving tube 300, so that the operator can pull the traction line 100 from the proximal end of the traction line 100, so that the traction line 100 drives the anchoring body 200 to flip.

It can be understood that the second length is equal to the difference between the total length of the traction line 100 and the first length. For example, the second length can be a suitable length such as 1/3 or 1/4 of the total length of the traction line 100. The embodiment of the present invention does not limit the first length and the second length, as long as it is ensured that the driving tube 300 can drive the traction line 100 and the anchoring body 200 to be smoothly implanted into the target tissue along the puncture needle 20, while the proximal end of the traction line 100 can be pulled by the operator, so as to make the anchoring body 200 flip over and anchor the target tissue on the outer wall of the first tissue.

Referring to Figures 1 to 7, the following is an exemplary description of the implantation process of the anchor 10, wherein, as an example, the first tissue is the intestine and the target tissue is the gallbladder.

After the puncture needle 20 is implanted in place, the traction line 100 and the anchoring body 200 at the distal end of the traction line 100 can be pushed from the proximal end of the puncture needle 20 into the needle channel of the puncture needle 20 through the driving tube 300.

The driving tube 300 is continued to be pushed to push the anchoring body 200 toward the distal end of the puncture needle 20 until the anchoring body 200 extends out of the distal end of the puncture needle 20 and the anchoring body 200 extends to the distal end of the driving tube 300, and then the traction line 100 is pulled to flip the anchoring body 200 to the anchoring angle.

It should be noted that in this step, the anchoring body 200 extending out of the distal end of the puncture needle 20 means that the entire part of the anchoring body 200 completely extends out of the distal end of the puncture needle 20, or a part of the anchoring body 200 extends out of the distal end of the puncture needle 20, as long as the traction line 100 is pulled so that the proximal end of the anchoring body 200 can move to the outside of the distal end of the puncture needle 20 during the flipping process.

The puncture needle 20 is withdrawn so that the puncture needle 20 is withdrawn into the instrument channel of the endoscope, and the driving tube 300 is withdrawn into the puncture needle 20; then the puncture needle 20 is withdrawn together with the driving tube 300, and the traction line 100 is left in the instrument channel.

The traction line 100 is pulled, for example, the traction line 100 can be pulled from the proximal end of the traction line 100, so that the anchoring body 200 is first attached to the inner wall of the gallbladder, and then the gallbladder is pulled to the outer wall of the intestine to achieve the anchoring of the gallbladder.

As shown in Figure 6 and Figure 7, in some examples, the driving tube 300 can be sleeved on the traction line 100, and the anchoring body 200 is located at the distal end of the driving tube 300, that is, the driving tube 300 is only sleeved on the traction line 100, and the anchoring body 200 is exposed outside the driving tube 300 and is located at the distal end of the driving tube 300.

When the anchoring body 200 is located in the puncture needle 20, the proximal end of the anchoring body 200 (as shown in 200a in Figure 5 and Figure 6 ) abuts against the distal end of the driving tube 300, so that when the anchoring body 200 extends out of the puncture needle 20, for example, when the proximal end of the anchoring body 200 is flush with the distal end of the puncture needle 10, the anchoring body 200 can be flipped under the pushing of the driving tube 300 and the pulling of the traction line 100.

As shown in Figures 6 and 7, for example, during the implantation of the anchor 10, the driving tube 300 can first push the distal end of the anchoring body 200 (as shown in 200b in Figures 5 to 7) from the proximal end of the puncture needle 20 to gradually enter the needle channel of the puncture needle 20, and then the distal end of the driving tube 300 abuts the proximal end of the anchoring body 200, so that the anchoring body 200 is pushed toward the distal end of the puncture needle 20, until the anchoring body 200 extends out of the distal end of the puncture needle 20, for example, when the proximal end of the anchoring body 200 just reaches the distal end of the puncture needle 20, the driving tube 300 can continue to abut the anchoring body 200, and the traction line 100 is pulled at the same time, so that the anchoring body 200 can achieve stable flipping under the assistance of the pushing of the driving tube 300 and the pulling of the traction line 100.

Of course, in some examples, when the anchoring body 200 extends out of the distal end of the puncture needle 20, the driving tube 300 may not be needed to abut the anchoring body 200, and the anchoring body 200 may be flipped under the pull of the traction line 100.

When the anchoring body 200 is located in the puncture needle 20, the length direction of the anchoring body 200 may be parallel to the extension direction of the puncture needle 20, or may have an angle with the extension direction of the puncture needle 20. However, because the traction line 100 is arranged as a flexible member, there is no resilience force between the traction line 100 and the anchoring body 200, so that the anchoring body 200 will not excessively abut against the side wall of the puncture needle 20 when moving in the puncture needle 20, and only move along the side wall of the puncture needle 20, reducing the friction between the anchoring body 200 and the puncture needle 20, ensuring that the anchoring body 200 moves stably along the puncture needle 20 under the drive of the driving tube 300, thereby improving the implantation stability and implantation efficiency of the anchoring body 200.

In some other examples, the driving tube 300 can be sleeved on the outer periphery of the traction line 100 and the anchoring body 200, that is, the anchoring body 200 and the traction line 100 are both located in the driving tube 300. During the implantation process of the anchor 10, the distal end of the driving tube 300 can gradually enter the needle channel of the puncture needle 20 from the proximal end of the puncture needle 20, so that the anchoring body 200 and a part of the traction line 100 enter the needle channel of the puncture needle 20, and then push the driving tube 300, so that the driving tube 300 is advanced toward the distal end of the puncture needle 20. When the anchoring body 200 reaches the distal end of the puncture needle 20 under the drive by the driving tube 300, for example, the distal end of the driving tube 300 is flush with the distal end of the anchoring body 200, and the distal end of the driving tube 300 just reaches the distal end of the puncture needle 20, it indicates that the distal end of the anchoring body 200 reaches the distal end of the puncture needle 20, and then the driving tube 300 can be withdrawn, so that the anchoring body 200 is exposed from the driving tube 300, and the proximal end of the anchoring body 200 can be abutted against the distal end of the driving tube 300. Then the driving tube 300 is continuously pushed toward the distal end of the puncture needle 20, so that the anchoring body 200 is exposed from the puncture needle 20 under the push of the driving tube 300. When the proximal end of the anchoring body 200 just reaches the distal end of the puncture needle 20, the driving tube 300 can continue to abut against the anchoring body 200, and at the same time the traction line 100 is pulled, so that the anchoring body 200 can achieve stable flipping under the assistance of the pushing of the driving tube 300 and the pulling of the traction line 100.

Of course, in some examples, a part of the anchoring body 200 can be arranged in the driving tube 300, and the other part can be exposed outside the driving tube 300. When the distal end of the anchoring body 200 reaches the distal end of the puncture needle 20, the driving tube 300 can be withdrawn so that the proximal end of the anchoring body 200 abuts against the distal end of the driving tube 300, and the driving tube 300 is continuously pushed toward the distal end of the puncture needle 20, so that the anchoring body 200 is exposed from the puncture needle 20 under the push of the driving tube 300. When the proximal end of the anchoring body 200 just reaches the distal end of the puncture needle 20, the driving tube 300 can continue to abut against the anchoring body 200, so that the anchoring body 200 can achieve stable flipping under the assistance of the pushing of the driving tube 300 and the pulling of the traction line 100.

It can be understood that in the above example, since the traction line 100 is a flexible member, there is no resilience force between the traction line 100 and the anchoring body 200, so that the anchoring body 200 will not be tightly against the side wall of the driving tube 300, so that the degree of contact between the anchoring body 200 and the inner wall of the driving tube 300 can allow the anchoring body 200 and the driving tube 300 to move relative to each other when the driving tube 300 is withdrawn. It is only necessary to ensure that the anchoring body 200 and the traction line 100 both can be pushed to the distal end of the puncture needle 20 during the advancement of the driving tube 300.

In addition, since the puncture needle 20 is implanted in advance, the anchoring body 200 of the embodiment of the present invention does not need to puncture the tissue, but only needs to move along the puncture needle 20 to the inside of the gallbladder, thereby avoiding the situation where the anchoring body 200 is implanted in the target tissue at an angle, so that the implantation efficiency and implantation success rate of the anchoring body 200 is further improved.

Compared with the above-mentioned example of the hard traction line 100, the arrangement of the flexible traction line 100 and the driving tube 300 can reduce the diameter of the traction line 100, thereby reducing the size of the traction line 100 occupied by the instrument channel of the endoscope, so that it is not necessary to withdraw the endoscope before implanting the fixator and re-implant it to the side of the traction line 100, thereby simplifying the surgical procedure and improving the surgical efficiency. In addition, there is no need to reduce the size of the fixator, thereby ensuring the installation efficiency and stability of the fixator, as well as the drainage effect of the fixator.

As an example, the diameter of the flexible traction line 100 can be 0.15mm, 0.1mm or 0.2mm, which is smaller than the diameter of the hard traction line 100. The embodiment of the present invention does not limit the diameter size of the traction line 100, as long as it is ensured that the traction line 100 can pull the anchoring body 200 to flip.

Referring to Figures 2b and 4, in some examples, the anchor 10 may include a distal locking structure 400. The distal locking structure 400 is configured to be fixed to the proximal end of the puncture needle 20. For example, the proximal end of the puncture needle 20 shown in Figure 2b may have a fixing portion 21, and the distal locking structure 400 may be fixed to the fixing portion 21 by means of internal and external thread matching, snap connection, flange connection, etc., to achieve the fixed connection between the anchor 10 and the proximal end of the puncture needle 20.

As an example, an inclined guide surface is formed at one end of the distal locking structure 400 that matches the fixing portion 21, so that the end of the distal locking structure 400 forms a guide portion 411 with a conical structure. During assembly, the guide portion 411 can be first extended into the fixing portion 21 to achieve the assembly pre-positioning between the distal locking structure 400 and the fixing portion 21, and then the distal locking structure 400 can be fixed to the fixing portion 21 by means of thread matching, etc.

Since the anchor 10 is long, the guide portion 411 can be arranged to preliminarily position the distal locking structure 400 on the fixing portion 21 of the puncture needle 20, so as to facilitate the rapid assembly and fixing of the distal locking structure 400 and the fixing portion 21, and avoid the situation where the anchor 10 shakes randomly and cannot be quickly assembled with the fixing portion 21.

As an example, a first receiving channel 430 is formed in the distal locking structure 400, and the first receiving channel 430 is connected to the needle channel of the puncture needle 20, and a part of the driving tube 300 and the anchoring body 200 are located in the first receiving channel 430. For example, the driving tube 300 and the anchoring body 200 located at the distal end of the driving tube 300 are located in the first receiving channel 430.

During the implantation of the anchor 10, the distal locking structure 400 can be first fixed to the fixing portion 21 at the proximal end of the puncture needle 20 to achieve the fixing of the anchor 10, and then the driving tube 300 can be pushed so that the driving tube 300 and the anchoring body 200 transition to the puncture needle 20 along the first receiving channel 430. The arrangement of the distal locking structure 400 can facilitate the driving tube 300 and the anchoring body 200 to align with the distal end of the puncture needle 20, and can quickly push the anchoring body 200 into the needle channel of the puncture needle 20, thereby improving the implantation efficiency of the anchor 10 into the puncture needle 20.

In some examples, the distal locking structure 400 can selectively cooperate with the driving tube 300 to lock the driving tube 300 and the anchoring body 200 in the first receiving channel 430, so that when the anchor 10 is docked with the proximal end of the puncture needle 20, the driving tube 300 can drive the anchoring body 200 to quickly enter the needle channel of the puncture needle 20. For example, before the anchoring body 200 is implanted into the puncture needle 20, the distal locking structure 400 can cooperate with the driving tube 300 so that the driving tube 300 and the anchoring body 200 are locked in the first receiving channel 430 to prevent the driving tube 300 from driving the anchoring body 200 to mistakenly enter into the first receiving channel 430 before the anchor 10 is fixed to the puncture needle 20, so that the anchoring body 200 is in a free state and cannot be quickly docked with the proximal end of the puncture needle 20.

In addition, when the anchoring body 200 is flipped to the anchoring angle, the puncture needle 20 and the driving tube 300 are withdrawn at the same time. In this process, the driving tube 300 can be locked on the puncture needle 20 through the cooperation between the distal locking structure 400 and the driving tube 300, so that the driving tube 300 can be withdrawn synchronously to the outside of the body during the withdrawal of the puncture needle 20, and the traction line 100 can be left in the instrument channel of the endoscope.

When the driving tube 300 needs to move relative to the puncture needle 20, the driving tube 300 can be released by the distal locking structure 400 so that the driving tube 300 can move along the puncture needle 20. For example, when the distal end of the puncture needle 20 reaches the preset position, the driving tube 300 can be released by the distal locking structure 400, so that the traction line 100 and the anchoring body 200 can be pushed toward the target tissue along the puncture needle 20 by pushing the driving tube 300 toward the puncture needle 20.

In some examples, the distal locking structure 400 can be a cylindrical structure, and the first receiving channel 430 is formed inside the cylindrical structure along the length direction of the cylindrical structure for the driving tube 300 and the anchoring body 200 to pass through.

For example, an elastic protrusion can be formed on the inner wall of the distal locking structure 400, and the elastic protrusion extends toward the first receiving channel 430. By making the elastic protrusion abuts against the outer wall of the driving tube 300, the driving tube 300 and the anchoring body 200 are locked on the distal locking structure 400. When the distal locking structure 400 is fixed to the fixing part 21 and the anchoring body 200 needs to be implanted, the elastic protrusion can be separated from the outer wall of the driving tube 300, so that the driving tube 300 and the anchoring body 200 are released from the distal locking structure 400, and the driving tube 300 can move along the puncture needle 20. By forming an elastic protrusion on the inner wall of the distal locking structure 400 to lock the driving tube 300, the structure and assembly process of the distal locking structure 400 can be simplified.

Continuing to refer to Figure 4, in some other examples, the distal locking structure 400 may include a distal connecting member 410 and a distal locking member 420, wherein the distal connecting member 410 is configured to be fixed to the proximal end of the puncture needle 20 and is provided with at least a portion of the first receiving channel 430 for the driving tube 300 and the anchoring body 200 to pass through. For example, the distal connecting member 410 is fixedly connected to the fixing portion 21 at the proximal end of the puncture needle 20.

The distal locking member 420 is disposed on the distal connecting member 410 and is configured to selectively cooperate with the driving tube 300 to lock or release the driving tube 300 and the anchoring body 200 on the distal connecting member 410.

As an example, when the distal locking member 420 cooperates with the driving tube 300, the driving tube 300 can be locked on the distal connecting member 410. When the distal locking member 420 is disengaged from the driving tube 300, the driving tube 300 can be released from the distal connecting member 410, so that the driving tube 300 can move relative to the distal connecting member 410, so that the driving tube 300 can push the anchoring body 200 along the first receiving channel 430 into the needle channel of the puncture needle 20.

In some examples, the distal locking member 420 can be a limit pin penetrated on the distal connecting member 410. For example, the distal connecting member 410 has a pin hole, and the limit pin is penetrated in the pin hole. After the distal connecting member 410 is fixedly connected to the fixing portion 21 of the puncture needle 20, the limit pin can be further pushed into the pin hole until one end of the limit pin abuts against the outer wall of the driving tube 300, so that the driving tube 300 and the anchoring body 200 can be locked. When the limit pin is pulled outward so that one end of the limit pin is disengaged from the driving tube 300, the driving tube 300 and the anchoring body 200 can be released.

In some other examples, the distal locking member 420 may include a distal fixing portion 421 and a distal locking portion 422. The distal fixing portion 421 is provided with a first receiving channel 430 and can be selectively fixed to or released from the distal connecting member 410. In other words, the distal fixing portion 421 can be fixed to the distal connecting member 410 or released from the distal connecting member 410.

The distal locking portion 422 is located in the distal fixing portion 421. When the distal fixing portion 421 is fixed to the distal connecting member 410, the distal locking portion 422 is configured to cooperate with the distal connecting member 410 and act on the driving tube 300 to lock the driving tube 300 and the anchoring body 200.

For example, the distal fixing portion 421 is of a cylindrical structure, which can be sleeved on a part of the structure of the distal connecting member 410 when fixed to the distal connecting member 410, and the distal locking portion 422 is located on the inner wall of the distal fixing portion 421, that is, on the inner wall of the first receiving channel 430.

As an example, the distal fixing portion 421 is of a hollow structure, and the end of the distal fixing portion 421 away from the distal connecting member 410 has a through hole (such as the first through hole 423) for the driving tube 300 to pass through, and the end of the distal fixing portion 421 facing the distal connecting member 410 is of an opening structure. The opening structure allows the distal connecting member 410 to enter the distal fixing portion 421 and connect with the distal fixing portion 421. It can be understood that the hollow inner cavity of the distal fixing portion 421 can be used as a part of the first receiving channel 430. When the distal fixing portion 421 is disengaged from the distal connecting member 410, the hollow inner cavity allows the driving tube 300 to pass through, and the first through hole 423 at the end of the distal fixing portion 421 is used as another part of the first receiving channel 430 to allow the proximal end of the driving tube 300 to extend out of the distal locking structure 400.

As an example, the distal fixing portion 421 can be a distal locking nut, which is threadedly connected to the distal connecting member 410 to simplify the connection structure between the distal fixing portion 421 and the distal connecting member 410, and it is also convenient to fix the distal locking portion 422 in the distal fixing portion 421 and facilitate the cooperation between the distal locking portion 422 and the distal connecting member 410.

When the distal fixing portion 421 is fixed to the distal connecting member 410, one end of the distal connecting member 410 extends into the distal fixing portion 421 and abuts against the distal locking portion 422. Under the abutment of the distal connecting member 410, one end of the distal locking portion 422 acts on the driving tube 300, for example, one end abuts against the driving tube 300 to lock the driving tube 300 and the anchoring body 200.

When the distal fixing portion 421 is released from the distal connecting member 410, the distal locking portion 422 is configured to be detached from the distal connecting member 410 to release the driving tube 300 and the anchoring body 200. For example, when the distal fixing portion 421 is released from the distal connecting member 410, one end of the distal connecting member 410 will be separated from the interior of the distal fixing portion 421, thereby separating from the distal locking portion 422. After the distal locking portion 422 is separated from the abutment of the distal connecting member 410, it can be restored to the original position, so that one end of the distal locking portion 422 is disengaged from the driving tube 300 to release the driving tube 300 and the anchoring body 200.

As an example, the distal locking portion 422 can be a lever (e.g., a first lever) disposed on the inner wall of the distal fixing portion 421, and the initial angle between the first lever and the inner wall of the distal fixing portion 421 is less than 90°, and the first lever is inclined toward the direction of the distal connecting member 410.

When the distal fixing portion 421 is fixed to the distal connecting member 410, one end of the distal connecting member 410 extends into the distal fixing portion 421 and abuts against the first lever. The first lever rotates under the abutment and push of the distal connecting member 410, so that the first lever rotates in a direction perpendicular to the inner wall of the distal fixing portion 421 until the free end of the first lever is on the driving tube 300, thereby the locking of the driving tube 300 can be achieved.

When the distal fixing portion 421 is detached from the distal connecting member 410, one end of the distal connecting member 410 will be separated from the interior of the distal fixing portion 421, thereby separating from the first lever. After the first lever is separated from the abutment of the distal connecting member 410, it can be rotated to the initial position, that is, the angle between the first lever and the inner wall of the distal fixing portion 421 is the initial angle, so that the free end of the first lever is separated from the driving tube 300, so as to achieve the release of the driving tube 300.

The embodiment of the present invention links the locking and releasing process of the distal locking portion 422 on the driving tube 300 with the fixing and releasing of the distal fixing portion 421 relative to the distal connecting member 410. For example, when the distal fixing portion 421 is fixed to the distal connecting member 410, the distal locking portion 422 will lock the driving tube 300 with the cooperation of the distal connecting member 410. When the distal fixing portion 421 is released from the distal connecting member 410, the distal locking portion 422 will release the driving tube 300 and the anchoring body 200. In this way, the operator can achieve the locking and releasing of the distal locking portion 422 on the driving tube 300 by operating the distal fixing portion 421, without performing other additional locking operations, thereby simplifying the locking and releasing process of the driving tube 300.

In some examples, the distal locking portion 422 may be a distal elastic member embedded in the distal fixing portion 421, and the distal elastic member is elastic at least along the first direction; and the first direction intersects with the extension direction of the driving tube 300. When the distal fixing portion 421 is fixed to the distal connecting member 410, the distal elastic member is pressed by the distal connecting member 410 and abuts against the driving tube 300 along the first direction to lock the driving tube 300 and the anchoring body 200. When the distal fixing portion 421 is released from the distal connecting member 410, the distal elastic member is disengaged from the distal connecting member 410, so as to release the driving tube 300 and the anchoring body 200 during the rebound process.

As an example, the distal elastic member is disposed between the inner wall of the distal fixing portion 421 and the driving tube 300, wherein the distal elastic member has a first end and a second end opposite to each other along the first direction, the first end abuts against the inner wall of the distal fixing portion 421, and the second end contacts the outer wall of the driving tube 300. It should be noted that the first direction may be perpendicular to the extension direction of the driving tube 300, or the angle between the first direction and the extension direction of the driving tube 300 may be an obtuse angle or an acute angle. The first direction may refer to the direction a in Figure 4. It is understood that the direction a may be the y direction, or may have an angle with the y direction.

It is understood that the angle between the first direction and the extension direction of the driving tube 300 refers to the angle between the first end of the distal elastic member and the driving tube 300 toward the distal connecting member 410.

Taking the first direction perpendicular to the extension direction of the driving tube 300 as an example, that is, when the first direction is the y direction, there is a certain gap between the part of the first end of the distal elastic member close to the distal connecting member 410 and the inner wall of the distal fixing portion 421. It is understood that in the initial state, when the second end of the distal elastic member contacts the driving tube 300, the driving tube 300 can move relative to the distal fixing portion 421, that is, in the initial state, the second end of the distal elastic member is only in simple contact with the driving tube 300, and the action force between the second end of the distal elastic member and the driving tube 300 is small.

When the distal fixing portion 421 is fixed to the distal connecting member 410, the distal connecting member 410 extends into the gap between the first end of the distal elastic member and the distal fixing portion 421, and the distal elastic member is pressed by the distal connecting member 410 and abuts the driving tube 300 along the first direction. For example, the distal connecting member 410 presses the distal elastic member along the first direction, so that the second end of the distal elastic member is in close contact with the driving tube 300, that is, the action force between the second end of the distal elastic member and the driving tube 300 is increased to lock the driving tube 300 and the anchoring body 200. When the distal fixing portion 421 is released from the distal connecting member 410, the distal elastic member is disengaged from the distal connecting member 410, and the distal elastic member is restored to the initial state, so that the action force between the second end of the distal elastic member and the driving tube 300 is reduced, that is, the distal elastic member releases the driving tube 300 and the anchoring body 200 during the rebound process.

Of course, in some examples, when the first direction is at an acute angle with the extension direction of the driving tube 300, and the distal fixing portion 421 is fixed to the distal connecting member 410, the distal connecting member 410 presses the distal elastic member along the first direction in a tilt manner, and when the distal elastic member is pressed by the distal connecting member 410, its second end abuts the driving tube 300 to lock the driving tube 300 and the anchoring body 200. When the distal fixing portion 421 is released from the distal connecting member 410, the distal elastic member is disengaged from the distal connecting member 410, and the distal elastic member is restored to the initial state along the first direction, so that the action force between the second end of the distal elastic member and the driving tube 300 is reduced, that is, the distal elastic member releases the driving tube 300 and the anchoring body 200 during the rebound process.

In some examples, the distal elastic member can be a spring, an elastic gasket, etc. When the distal elastic member is a structure such as a spring or an elastic gasket, the distal elastic member may be in a number of one or more. When there is one distal elastic member, the distal elastic member is arranged at any position along the circumference of the distal fixing portion 421. When there are multiple distal elastic members, the multiple distal elastic members may be arranged at intervals along the circumference of the inner wall of the distal fixing portion 421, that is, the multiple distal elastic members are arranged at intervals around the circumference of the driving tube 300, so that the second ends of the multiple distal elastic members can abut the driving tube 300 at multiple positions along the circumference when pressed by the distal connecting member 410, thereby a stable locking of the driving tube 300 can be achieved.

By arranging the distal locking member 420 as a distal elastic member, on the one hand, it is convenient to assemble the distal locking member 420 between the distal fixing portion 421 and the driving tube 300, and on the other hand, the distal locking member 420 can be reliably matched with the distal connecting member 410, and deformed accordingly, thereby the reliability of locking the driving tube 300 can be improved. In addition, the distal locking member 420 is arranged as a distal elastic member, so that the distal locking member 420 can smoothly rebound to the original position after being separated from the distal connecting member 410, thereby the release reliability of the driving tube 300 is improved.

In some examples, the distal elastic member can be a distal elastic sleeve. The distal elastic sleeve is provided with a first penetration hole for the driving tube 300 to pass through. For example, the distal elastic sleeve is sleeved on a part of the outer wall of the driving tube 300, and the distal elastic sleeve is received in the end inner cavity of the distal fixing portion 421.

When the distal elastic sleeve is in the initial state, that is, when it is not matched with the distal connecting member 410, the hole wall of the first penetration hole is only in simple contact with the driving tube 300, and the driving tube 300 can move relatively in the first penetration hole.

When the distal connecting member 410 enters the distal fixing portion 421 and presses the distal elastic sleeve, the hole wall of the first penetration hole of the distal elastic sleeve abuts the driving tube 300 under the pressing by the distal connecting member 410 to lock the driving tube 300 and the anchoring body 200, and as the distal elastic sleeve is separated from distal connecting member 410, the hole wall of the first through hole rebounds to release the driving tube 300 and the anchoring body 200.

For example, when the distal fixing portion 421 is fixed to the distal connecting member 410, the distal connecting member 410 presses the distal elastic sleeve along the first direction. After being pressed by the distal connecting member 410, the distal elastic sleeve is deformed or compressed at least along the first direction, and the hole wall of the first penetration hole thereof abuts the driving tube 300, so that the action force between the hole wall of the first penetration hole and the driving tube 300 increases, so as to lock the driving tube 300 and the anchoring body 200. When the distal fixing portion 421 is released from the distal connecting member 410, the distal elastic sleeve is disengaged from the distal connecting member 410, and the distal elastic sleeve is restored to the initial state, so that the action force between the hole wall of the first penetration hole and the driving tube 300 is reduced, that is, the distal elastic sleeve releases the driving tube 300 and the anchoring body 200 during the rebound process.

By arranging the distal elastic member as the distal elastic sleeve, after the distal elastic sleeve is pressed by the distal connecting member 410, it can abut the entire surface of the driving tube 300 along the circumferential direction, thereby a stable locking of the driving tube 300 is achieved.

As an example, the distal elastic sleeve may include a sleeve structure with elastic deformation, such as a silicone sleeve, and the embodiment of the present invention does not limit the material of the distal elastic sleeve.

As shown in Figure 4, for example, the distal elastic sleeve may be an elastic ball, so that the outer surface of the elastic ball can be uniformly pressed and deformed by the circumferential end wall of the distal connecting member 410, so that the action forces at the inner wall of the first penetration hole and at each position of the circumference of the driving tube 300 are the same, thereby ensuring that the driving tube 300 is uniformly stressed without tilting.

Of course, in some examples, the distal elastic sleeve may also be a square or irregular sleeve structure, and the embodiment of the present invention does not limit the shape of the distal elastic sleeve.

Figure 8 is a sectional view of the proximal locking structure in Figure 3. As shown in Figure 3 and Figure 8, in some examples, the anchor 10 may also include a proximal locking structure 500.

The proximal locking structure 500 is disposed at the proximal end of the driving tube 300 and is fixedly connected to the driving tube 300. For example, the proximal locking structure 500 can be fixedly connected to the driving tube 300 by welding, bonding, snapping, etc., and the embodiment of the present invention does not limit the connection way between the proximal locking structure 500 and the driving tube 300.

In some examples, a second receiving channel 530 is formed in the proximal locking structure 500, and the traction line 100 is inserted into the second receiving channel 530. As an example, a part of the second receiving channel 530 can directly be penetrated by the traction line 100 exposed on the proximal end of the driving tube 300, and the other part can receive the proximal part of the driving tube 300 and be fixedly connected to the driving tube 300.

Because the traction line 100 of the embodiment of the present invention is a flexible member, during the driving tube 300 drives the flexible member and the anchoring body 200 to advance toward the distal end of the puncture needle 20, the proximal locking structure 500 is configured to selectively cooperate with the traction line 100 to lock the traction line 100 on the driving tube 300.

It can be understood that since the proximal end of the driving tube 300 is fixed to the proximal locking structure 500, the traction line 100 is locked in the proximal locking structure 500, so that the traction line 100 is locked on the driving tube 300.

For example, the distal end of the traction line 100 is fixed to the anchoring body 200, the anchoring body 200 is arranged on the distal end of the driving tube 300, and the proximal end of the anchoring body 200 abuts the distal end of the driving tube 300. When the traction line 100 does not need to move relative to the driving tube 300, the traction line 100 can be tightened in the driving tube 300, and the proximal locking structure 500 cooperates with the traction line 100 to tighten and lock the traction line 100 on the driving tube 300, so that the traction line 100 can be prevented from moving relative to the driving tube 300.

For example, before the driving tube 300 carries the traction line 100 and the anchoring body 200 to move into the puncture needle 20, the traction line 100 can be tightened and locked on the driving tube 300 through the proximal locking structure 500, and then the driving tube 300 can drive the traction line 100 and the anchoring body 200 to move synchronously along the puncture needle 20 into the target tissue. In this way, the traction line 100 can be prevented from accumulating in the puncture needle 20 during the process of the driving tube 300 delivering the anchoring body 200.

For example, when the anchoring body 200 is accidentally withdrawn during delivery and then continues to be pushed forward, the traction line 100 tightened by the proximal locking structure 500 can relieve or avoid accumulation between the distal end of the driving tube 300 and the anchoring body 200, compared with the traction line 100 without tension, thereby avoiding the accumulated and entangled traction line 100 from affecting the smooth pushing of the anchoring body 200 by the driving tube 300. In addition, it also avoids affecting the normal flipping of the anchoring body 200 during the subsequent pull back process of the traction line 100.

When the anchoring body 200 needs to move in place and needs to be flipped, for example, as the proximal end of the anchoring body 200 moves to the distal end of the puncture needle 20, the proximal locking structure 500 can release the traction line 100 so that the traction line 100 can move relative to the driving tube 300. In this way, the anchoring body 200 can be flipped by the pulling of the traction line 100.

In some examples, the proximal locking structure 500 may be of a cylindrical structure, and the second receiving channel 530 is formed inside the cylindrical structure along the length direction of the cylindrical structure for the traction line 100 to pass through. For example, an elastic protrusion may be formed on the inner wall of the proximal locking structure 500, and the elastic protrusion extends toward the second receiving channel 530.

When the proximal locking structure 500 is fixed to the proximal end of the driving tube 300, the elastic protrusion abuts the outer wall of the traction line 100, so that the traction line 100 is locked on the proximal locking structure 500. Since the proximal locking structure 500 is fixed to the driving tube 300, the traction line 100 is locked on the driving tube 300 and cannot move relative to the driving tube 300. When the proximal locking structure 500 is released from the driving tube 300, that is, after being detached from the driving tube 300, the elastic protrusion can be separated from the outer wall of the traction line 100, so that the traction line 100 is released from the proximal locking structure 500, thereby making the traction line 100 released from the driving tube 300 and move relatively along the driving tube 300. By forming an elastic protrusion on the inner wall of the proximal locking structure 500 to lock the traction line 100, the structure and assembly process of the proximal locking structure 500 can be simplified.

Continuing to refer to Figure 8, in some other examples, the proximal locking structure 500 includes a proximal connecting member 510 and a proximal locking member 520, wherein the proximal connecting member 510 is fixed to the proximal end of the driving tube 300, and at least part of the second receiving channel 530 is formed for the traction line 100 to pass through. As an example, the proximal part of the driving tube 300 can also be fixed in the second receiving channel 530, and the part of the traction line 100 extending out from the proximal end of the driving tube 300 is passed through the other parts of the second receiving channel 530.

The proximal locking member 520 is disposed on the proximal connecting member 510 and is configured to selectively cooperate with the traction line 100 to lock or release the traction line 100 on the proximal connecting member 510.

For example, when the proximal locking member 520 cooperates with the traction line 100, the traction line 100 can be locked on the proximal connecting member 510, and the proximal connecting member 510 is fixed to the proximal end of the driving tube 300, then the traction line 100 is locked on the driving tube 300, so that the traction line 100 is tightened in the driving tube 300 and cannot move relative to the driving tube 300. When the proximal locking member 520 is disengaged from the proximal connecting member 510, the traction line 100 can be released on the proximal connecting member 510, that is, released on the driving tube 300, so that the traction line 100 can move relative to the driving tube 300.

In some examples, the proximal locking member 520 may be a stop pin inserted into the proximal connecting member 510. For example, the proximal connecting member 510 has a pin hole, and the stop pin is inserted into the pin hole. After the proximal connecting member 510 is fixedly connected to the proximal end of the driving tube 300, the stop pin can be further pushed into the pin hole until one end of the stop pin abuts the outer wall of the traction line 100, thereby locking the traction line 100. When the stop pin is pulled outward so that one end of the stop pin is separated from the traction line 100, the traction line 100 can be released.

In other examples, the proximal locking member 520 may include a proximal fixing portion 521 and a proximal locking portion 522. The proximal fixing portion 521 is provided with a second receiving channel 530, and can be selectively fixed to or released on the proximal connecting member 510. In other words, the proximal fixing portion 521 can be fixed on the proximal connecting member 510, and can also be released from the proximal connecting member 510.

The proximal locking portion 522 is located in the proximal fixing portion 521. When the proximal fixing portion 521 is fixed on the proximal connecting member 510, the proximal locking portion 522 is configured to cooperate with the proximal connecting member 510 and act on the traction line 100 to lock the traction line 100. For example, the proximal fixing portion 521 is of a cylindrical structure. When the cylindrical structure is fixed to the proximal connecting member 510, it can be sleeved on a part of the structure of the proximal connecting member 510. The proximal locking portion 522 is located on the inner wall of the proximal fixing portion 521, that is, on the inner wall of the second receiving channel 530.

As an example, the proximal fixing portion 521 is of a hollow structure, and the end of the proximal fixing portion 521 close to the proximal connecting member 510 has a through hole (such as a second through hole 523) for the traction line 100 to pass through, and the end of the proximal fixing portion 521 facing the proximal connecting member 510 is of an opening structure, and the opening structure allows the proximal connecting member 510 to enter the proximal fixing portion 521 and connect with the proximal fixing portion 521. It can be understood that the hollow inner cavity of the proximal fixing portion 521 can be used as a part of the second receiving channel 530. When the proximal fixing portion 521 is detached from the proximal connecting member 510, the hollow inner cavity allows the traction line 100 to pass through. The second through hole 523 at the end of the proximal fixing portion 521 serves as another part of the second receiving channel 530, so that the proximal end of the traction line 100 can extend out of the proximal locking structure 500.

As an example, the proximal fixing portion 521 may be a proximal locking nut, which is threadedly connected to the proximal connecting member 510 to simplify the connection structure between the proximal fixing portion 521 and the proximal connecting member 510, and to facilitate fixing the proximal locking portion 522 in the proximal fixing portion 521 and to facilitate the proximal locking portion 522 to cooperate with the proximal connecting member 510.

When the proximal fixing portion 521 is fixed to the proximal connecting member 510, one end of the proximal connecting member 510 extends into the proximal fixing portion 521 and abuts the proximal locking portion 522. Under the abutment of the proximal connecting member 510, one end of the proximal locking portion 522 acts on the traction line 100, for example, one end of the proximal locking portion abuts against the traction line 100 to lock the traction line 100.

When the proximal fixing portion 521 is released from the proximal connecting member 510, the proximal locking portion 522 is configured to be detached from the proximal connecting member 510 to release the traction line 100.

For example, when the proximal fixing portion 521 is detached from the proximal connecting member 510, one end of the proximal connecting member 510 will be detached from the interior of the proximal fixing portion 521, thereby detaching from the proximal locking portion 522. After the proximal locking portion 522 is detached from the abutment of the proximal connecting member 510, it can be restored to the original position, so that one end of the proximal locking portion 522 is detached from the traction line 100 to release the traction line 100.

As an example, the proximal locking portion 522 can be a lever (e.g., a second lever) disposed on the inner wall of the proximal fixing portion 521, and the initial angle between the second lever and the inner wall of the proximal fixing portion 521 is less than 90°, and the second lever is inclined in a direction toward the proximal connecting member 510.

When the proximal fixing portion 521 is fixed to the proximal connecting member 510, one end of the proximal connecting member 510 extends into the proximal fixing portion 521 and abuts the second lever. The second lever rotates under the abutment and push of the proximal connecting member 510, so that the second lever rotates in a direction perpendicular to the inner wall of the proximal fixing portion 521 until the free end of the second lever is on the traction line 100, thereby locking the traction line 100.

When the proximal fixing portion 521 is detached from the proximal connecting member 510, one end of the proximal connecting member 510 will be detached from the inside of the proximal fixing portion 521, thereby detached from the second lever. After the second lever is separated from the abutment of the proximal connecting member 510, it can be rotated to the initial position, that is, the angle between the second lever and the inner wall of the proximal fixing portion 521 is the initial angle, so that the free end of the second lever is detached from the traction line 100, so as to release the traction line 100.

The embodiment of the present invention links the locking and releasing process of the traction line 100 by the proximal locking portion 522 with the fixing and releasing of the proximal fixing portion 521 relative to the proximal connecting member 510. For example, when the proximal fixing portion 521 is fixed to the proximal connecting member 510, the proximal locking portion 522 will lock the traction line 100 with the cooperation of the proximal connecting member 510. When the proximal fixing portion 521 is released from the proximal connecting member 510, the proximal locking portion 522 will release the traction line 100. In this way, the operator can realize the locking and releasing of the traction line 100 by the proximal locking portion 522 by operating the proximal fixing portion 521 without performing other additional locking operations, thereby simplifying the locking and releasing process of the traction line 100.

In some examples, the proximal locking portion 522 may be a proximal elastic member embedded in the proximal fixing portion 521, and the proximal elastic member is elastic at least along the first direction; and the first direction intersects with the extension direction of the traction line 100.

When the proximal fixing portion 521 is fixed to the proximal connecting member 510, the proximal elastic member is pressed by the proximal connecting member 510 and abuts against the traction line 100 along the first direction to lock the traction line 100.

When the proximal fixing portion 521 is released from the proximal connecting member 510, the proximal elastic member is separated from the proximal connecting member 510 to release the traction line 100 during the rebound process.

As an example, the proximal elastic member is arranged between the inner wall of the proximal fixing portion 521 and the traction line 100, wherein the proximal elastic member has a first end and a second end opposite to each other along the first direction, the first end abuts against the inner wall of the proximal fixing portion 521, and the second end contacts the outer wall of the traction line 100. It should be noted that the first direction can be perpendicular to the extension direction of the traction line 100, or the angle between the first direction and the extension direction of the traction line 100 can be an obtuse angle or an acute angle. It can be understood that the angle between the first direction and the extension direction of the traction line 100 refers to the angle between the first end of the proximal elastic member and the traction line 100 toward the proximal connecting member 510.

Taking the first direction perpendicular to the extension direction of the traction line 100 as an example, there is a certain gap between the part of the first end of the proximal elastic member close to the proximal connecting member 510 and the inner wall of the proximal fixing portion 521. It can be understood that in the initial state, when the second end of the proximal elastic member contacts the traction line 100, the traction line 100 can move relative to the proximal fixing portion 521, that is, in the initial state, the second end of the proximal elastic member is only in simple contact with the traction line 100, and the action force between the second end of the proximal elastic member and the traction line 100 is small.

When the proximal fixing portion 521 is fixed to the proximal connecting member 510, the proximal connecting member 510 extends into the gap between the first end of the proximal elastic member and the proximal fixing portion 521, and the proximal elastic member is pressed by the proximal connecting member 510 and abuts the traction line 100 along the first direction. For example, the proximal connecting member 510 presses the proximal elastic member along the first direction, so that the second end of the proximal elastic member is in close contact with the traction line 100, that is, the action force between the second end of the proximal elastic member and the traction line 100 is increased to lock the traction line 100, When the proximal fixing portion 521 is released from the proximal connecting member 510, the proximal elastic member is separated from the proximal connecting member 510, and the proximal elastic member returns to the initial state, so that the action force between the second end of the proximal elastic member and the traction line 100 is reduced, that is, the proximal elastic member releases the traction line 100 during the rebound process.

Of course, in some examples, when the first direction and the extension direction of the traction line 100 are at an acute angle, and the proximal fixing portion 521 is fixed to the proximal connecting member 510, the proximal connecting member 510 tilts and presses the proximal elastic member along the first direction, and the second end of the proximal elastic member abuts the traction line 100 under the pressing by the proximal connecting member 510 to lock the traction line 100. When the proximal fixing portion 521 is released from the proximal connecting member 510, the proximal elastic member is separated from the proximal connecting member 510, and the proximal elastic member is restored to the initial state along the first direction, so that the action force between the second end of the proximal elastic member and the traction line 100 is reduced, that is, the proximal elastic member releases the traction line 100 during the rebound process.

In some examples, the proximal elastic member can be a spring, an elastic gasket, etc. When the proximal elastic member is a structure such as a spring or an elastic gasket, the proximal elastic member can be in a number of one or more. When there is one proximal elastic member, the proximal elastic member is arranged at any position of the proximal fixing portion 521 along the circumference. When there are multiple proximal elastic members, the multiple proximal elastic members can be arranged at intervals on the inner wall of the proximal fixing portion 521 along the circumference, that is, the multiple proximal elastic members are arranged at intervals on the traction line 100 around the circumference direction, so that the second ends of the multiple proximal elastic members can abut the traction line 100 at multiple positions along the circumference when pressed by the proximal connecting member 510, thereby achieving stable locking of the traction line 100.

By arranging the proximal locking member 520 as a proximal elastic member, on the one hand, it is convenient to assemble the proximal locking member 520 between the proximal fixing portion 521 and the traction line 100, and on the other hand, the proximal locking member 520 can be reliably matched with the proximal connecting member 510 and deformed accordingly, thereby improving the reliability of locking the traction line 100. In addition, the proximal locking member 520 is arranged as a proximal elastic member, so that the proximal locking member 520 can smoothly rebound to the original position after being separated from the proximal connecting member 510, thereby improving the release reliability of the traction line 100.

In some examples, the proximal elastic member can be a proximal elastic sleeve.

The proximal elastic sleeve is provided with a second penetration hole for the traction line 100 to pass through.

For example, the proximal elastic sleeve is sleeved on a part of the outer wall of the traction line 100, and the proximal elastic sleeve is received in the end inner cavity of the proximal fixing portion 521 relative to the opening structure.

When the proximal elastic sleeve is in the initial state, that is, when it is not matched with the proximal connecting member 510, the hole wall of the second penetration hole is only in simple contact with the traction line 100, and the traction line 100 can move relatively in the second penetration hole.

When the proximal connecting member 510 enters the proximal fixing portion 521 and presses the proximal elastic sleeve, the hole wall of the second penetration hole of the proximal elastic sleeve abuts the traction line 100 under the pressing by the proximal connecting member 510 to lock the traction line 100, and after the proximal elastic sleeve is separated from the proximal connecting member 510, the hole wall of the penetration hole rebounds to release the traction line 100.

For example, when the proximal fixing portion 521 is fixed to the proximal connecting member 510, the proximal connecting member 510 presses the proximal elastic sleeve along the first direction. After being pressed by the proximal connecting member 510, the proximal elastic sleeve is deformed and compressed at least along the first direction, and the hole wall of the second penetration hole abuts the traction line 100, so that the action force between the hole wall of the second penetration hole and the traction line 100 increases to lock the traction line 100. When the proximal fixing portion 521 is released from the proximal connecting member 510, the proximal elastic sleeve is separated from the proximal connecting member 510, and the proximal elastic sleeve returns to the initial state, so that the action force between the hole wall of the second penetration hole and the traction line 100 decreases, that is, the proximal elastic sleeve releases the traction line 100 during the rebound process.

By arranging the proximal elastic member as the proximal elastic sleeve, after the proximal elastic sleeve is pressed by the proximal connecting member 510, it can abut the entire surface of the traction line 100 along the circumferential direction, thereby achieving stable locking of the traction line 100.

For example, the proximal elastic sleeve may include a sleeve structure with elastic deformation, such as a silicone sleeve, and the embodiment of the present invention does not limit the material of the proximal elastic sleeve.

For example, the proximal elastic sleeve may be an elastic ball, so that the outer surface of the elastic ball can be uniformly pressed and deformed by the circumferential end wall of the proximal connecting member 510, so that the inner wall of the second penetration hole and any position along the circumference of the traction line 100 have the same action force, thereby ensuring that the traction line 100 is uniformly stressed without tilting.

Of course, in some examples, the proximal elastic sleeve may also be a square or irregular sleeve structure, and the embodiment of the present invention does not limit the shape of the proximal elastic sleeve.

The following continues to take the intestine as the first tissue and the gallbladder as the target tissue as an example to illustrate the implantation process of the anchoring device.

The distal connecting member 410 of the distal locking structure 400 is fixed to the fixing portion 21 at the proximal end of the puncture needle 20, and the distal fixing portion 421 is fixed to the distal connecting member 410. The driving tube 300 is locked in the distal connecting member 410, and the puncture needle 20 with the anchor 10 at the proximal end moves toward the target tissue until the puncture needle 20 is implanted in place, and then the distal fixing portion 421 is detached from the distal connecting member 410, and the driving tube 300 is released from the distal connecting member 410.

The proximal fixing portion 521 is fixed on the proximal connecting member 510 to tighten the traction line 100 and push the driving tube 300 to move into the needle channel of the puncture needle 20, so as to push the traction line 100 and the anchoring body 200 at the distal end of the traction line 100 from the proximal end of the puncture needle 20 into the needle channel of the puncture needle 20.

The driving tube 300 is continued to be pushed so that the anchoring body 200 is pushed toward the distal end of the puncture needle 20 until the anchoring body 200 extends out of the distal end of the puncture needle 20. For example, the proximal end of the anchoring body 200 is flush with the distal end of the puncture needle 20, and the proximal end of the anchoring body 200 abuts against the distal end of the driving tube 300, and the proximal fixing portion 521 is detached from the proximal connecting member 510 to release the traction line 100, and then the traction line 100 is pulled to flip the anchoring body 200 to the anchoring angle.

The puncture needle 20 is withdrawn so that the puncture needle 20 is withdrawn into the instrument channel of the endoscope, and the driving tube 300 is withdrawn into the puncture needle 20; then the distal fixing portion 421 is fixed to the distal connecting member 410 to lock the driving tube 300 at the proximal end of the puncture needle 20, and the puncture needle 20 is withdrawn together with the driving tube 300, leaving the traction line 100 in the instrument channel.

The traction line 100 is pulled, for example, from the proximal end of the traction line 100, so that the anchoring body 200 is first attached to the inner wall of the gallbladder, and then the gallbladder is pulled to the outer wall of the intestine to achieve the anchoring of the gallbladder.

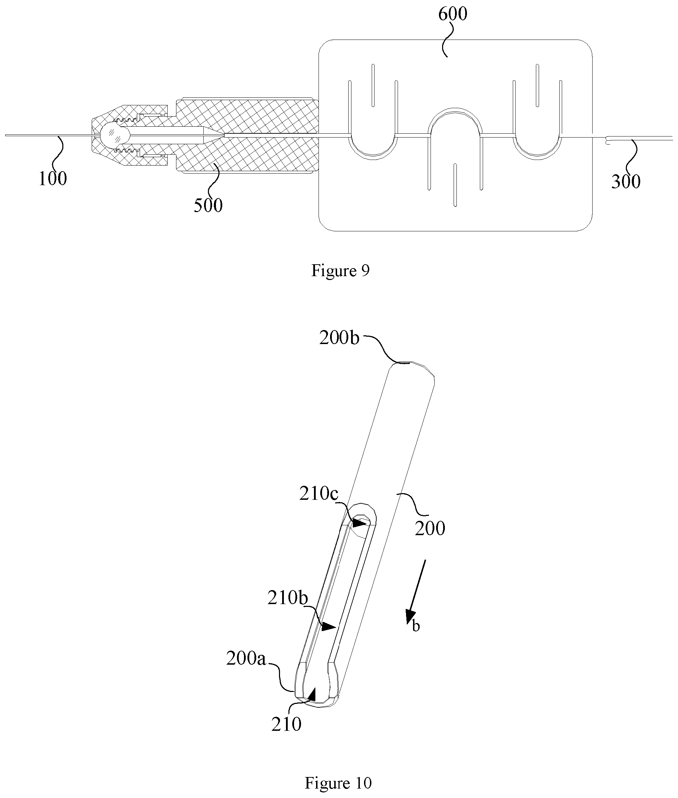

Figure 9 is a schematic diagram of the cooperation between the proximal locking structure and the stopper in Figure 3. As shown in Figure 3 and Figure 9, in some examples, the anchor 10 may further include a stopper 600.

The stopper 600 is selectively disposed between the driving tube 300 and the puncture needle 20. In other words, the stopper 600 may be located between the driving tube 300 and the puncture needle 20, or may be detached from the position between the driving tube 300 and the puncture needle 20. For example, the stopper 600 in one state is disposed between the driving tube 300 and the puncture needle 20, and in another state detached from the driving tube 300 and the puncture needle 20.

When the anchoring body 200 is located in the puncture needle 20 and has a preset distance from the distal end of the puncture needle 20, the stopper 600 cooperates with the driving tube 300 and the puncture needle 20 respectively to limit the movement of the driving tube 300 in the puncture needle 20.

During the movement of the anchoring body 200 from the first position to the distal end of the puncture needle 20, the stopper 600 is detached from the position between the driving tube 300 and the puncture needle 20, wherein the first position is the position when the anchoring body 200 has a preset distance from the distal end of the puncture needle 20.

For example, the stopper 600 may include an elastic snap and a slot, wherein the elastic snap is provided on one of the driving tube 300 and the puncture needle 20, and the slot is provided on the other one of the driving tube 300 and the puncture needle 20.

When the anchoring body 200 is located in the puncture needle 20 and has a preset distance from the distal end of the puncture needle 20, the elastic snap is snapped into the slot to limit the movement of the driving tube 300 in the puncture needle 20.

During the movement of the anchoring body 200 from the first position to the distal end of the puncture needle 20, the elastic snap is disengaged from the slot, and the driving tube 300 can continue to move along the puncture needle 20.

For example, the preset distance can be 0, that is, the distal end of the anchoring body 200 is flush with the distal end of the puncture needle 20; the preset distance can also be any value less than or equal to 10mm, for example, the preset distance can be a suitable value such as 2mm, 4mm, 7mm or 10mm. Of course, the preset distance is only to express the distance closer to the distal end of the puncture needle 20, and the embodiment of the present invention does not limit the preset distance.

By disposing a stopper 600 between the driving tube 300 and the puncture needle 20, when the anchoring body 200 moves along the needle channel of the puncture needle 20 to a preset distance from the distal end of the puncture needle 20, the driving tube 300 can be stopped to prompt the operator that the anchoring body 200 is about to reach the distal end of the puncture needle 20 or has reached the distal end of the puncture needle 20, and then the advancing speed of the driving tube 300 can be controlled to make the anchoring body 200 slowly approach the distal end of the puncture needle 20 or slowly extend out of the distal end of the puncture needle 20, thereby ensuring that when the proximal end of the anchoring body 200 approaches the distal end of the puncture needle 20 or reaches the distal end of the puncture needle 20, the traction line 100 can be pulled back in time to make the anchoring body 200 flip in time, and avoiding the anchoring body 200 extending too far from the distal end of the puncture needle 20, which affects the flipping of the anchoring body 200 or causes damage to the target tissue.

In addition, when the stopper 600 does not lock the driving tube 300 on the puncture needle 20, that is, before the anchoring body 200 reaches the first position, the advancing speed of the driving tube 300 can be accelerated to improve the implantation efficiency of the anchor 10.

In the specific arrangement, the stopper 600 can be disposed at any position of the driving tube 300 along the length direction. For example, the stopper 600 is disposed between the proximal end of the driving tube 300 and the proximal end of the puncture needle 20, and selectively protrudes from the side wall of the driving tube 300.

At least when the anchoring body 200 is located in the puncture needle 20 and has a preset distance from the distal end of the puncture needle 20, the stopper 600 protrudes from the side wall of the driving tube 300, so that the proximal end of the driving tube 300 is blocked by the stopper 600 at the proximal end of the puncture needle 20. For example, when the anchoring body 200 is located in the puncture needle 20 and has a preset distance with the distal end of the puncture needle 20, the stopper 600 protrudes from the side wall of the driving tube 300; or when the distance between the anchoring body 200 and the distal end of the puncture needle 20 is greater than the preset distance, the stopper 600 protrudes from the side wall of the driving tube 300, that is, before the anchoring body 200 moves from the proximal end of the puncture needle 20 to the first position, the stopper 600 may always protrude from the side wall of the driving tube 300.

As an example, the stopper 600 is disposed between the proximal locking structure 500 and the distal locking structure 400, and when the stopper 600 protrudes from the side wall of the driving tube 300, and the stopper 600 moves with the driving tube 300 to one end of the distal locking structure 400, the stopper 600 may be blocked on one side of the distal locking structure 400 (i.e., the side facing the proximal locking structure 500).

It can be understood that when arranging, the outer diameter of the driving tube 300 can be adapted to the first through hole 423 of the distal locking structure 400. When the stopper 600 protrudes from the side wall of the driving tube 300, the radial distance between the outer end of the stopper 600 and the driving tube 300 is greater than the radius of the first through hole 423, so that the stopper 600 is blocked on one side of the distal locking structure 400, that is, the proximal end of the driving tube 300 is blocked on the proximal end of the puncture needle 20 to prevent the driving tube 300 from continuing to advance along the puncture needle 20.

In addition, during the movement of the anchoring body 200 from the first position to the distal end of the puncture needle 20, the stopper 600 is detached from the side wall of the driving tube 300. In other words, when the stopper 600 reaches one side of the distal locking structure 400, there is a preset distance between the distal end of the anchoring body 200 and the distal end of the puncture needle 20. Then, the stopper 600 is detached from the side wall of the driving tube 300, so that the driving tube 300 continues to slowly advance into the puncture needle 20, so that the anchoring body 200 extends out of the distal end of the puncture needle 20 and flips.

In some examples, the stopper 600 can be an elastic protrusion, which can extend out of the side wall of the driving tube 300 or retract into the driving tube 300. For example, a receiving groove can be formed on the driving tube 300, and the distal end of the elastic protrusion is fixed in the receiving groove. In a normal state, the elastic protrusion protrudes from the receiving groove, and when the elastic protrusion is pressed laterally or vertically, it can be retracted into the receiving groove.

For example, when the anchoring body 200 is located in the puncture needle 20 and before reaching the first position, the elastic protrusion protrudes from the side wall of the driving tube 300, so that when the anchoring body 200 reaches the first position, the elastic protrusion is arranged to block on one side of the distal locking structure 400. When the driving tube 300 continues to be pushed into the puncture needle 20, the elastic protrusion retracts into the receiving groove under the pressing of the inner wall of the distal locking structure 400, so that the driving tube 300 is smoothly pushed to the puncture needle 20.

In some other examples, the stopper 600 can be detachably arranged on the driving tube 300. When the anchoring body 200 moves from the first position to the distal end of the puncture needle 20, the stopper 600 is configured to be detached from the driving tube 300. In this way, the assembly process between the stopper 600 and the driving tube 300 can be simplified, making the process control of the stopper 600 being detached from the side wall of the driving tube 300 more convenient and quick. In addition, the integrity of the structure of the driving tube 300 itself does not need to be destroyed, so that the strength of the driving tube 300 will not be affected.

As an example, the stopper 600 can be a limit splint, which clamps the driving tube 300. When the anchoring body 200 reaches the first position and continues to move toward the distal end of the puncture needle 20, the limit splint can be removed from the driving tube 300, so that the driving tube 300 continues to advance along the puncture needle 20.

In some examples, the stopper 600 can be disposed at one end of the proximal locking structure 500 of the anchor 10 toward the distal locking structure 400. In this example, the preset distance is 0, that is, when the anchoring body 200 is in the first position, its distal end is just flush with the distal end of the puncture needle 20. The length of the stopper 600 is equal to the maximum length of the anchoring body 200 extending out from the distal end of the puncture needle 20.

In this way, when the stopper 600 arrives at one end of the distal locking structure 400 toward the proximal locking structure 500 along with the driving tube 300, the proximal locking structure 500 and the distal locking structure 400 are just separated by the stopper 600, and at this time, the distal end of the anchoring body 200 is flush with the distal end of the puncture needle 20. After removing the stopper 600, the driving tube 300 continues to advance toward the puncture needle 20. When the proximal locking structure 500 moves to one end of the distal locking structure 400, the movement distance of the driving tube 300 is the length of the stopper 600, and the movement distance of the anchoring body 200 is also the length of the stopper 600, so that the length of the anchoring body 200 extending out from the distal end of the puncture needle 20 is the length of the stopper 600.

By arranging the stopper 600 at one end of the proximal locking structure 500 facing the distal locking structure 400, the operator can intuitively determine the maximum length of the anchoring body 200 extending out from the distal end of the puncture needle 20 according to the length of the stopper 600, thereby providing a reliable basis for the subsequent pull-back distance of the traction line 100.

For example, the length of the stopper 600 can be equal to the length of the anchoring body 200, so that when the proximal locking structure 500 moves to the end of the distal locking structure 400, the proximal end of the anchoring body 200 is just flush with the distal end of the puncture needle 20, that is, the length of the anchoring body 200 extending out from the distal end of the puncture needle 20 is equal to the entire length of the anchoring body 200. In this way, when the proximal locking structure 500 moves to the end of the distal locking structure 400, the proximal fixing portion 521 can be removed from the proximal connecting member 510 to release the traction line 100, and then the traction line 100 is pulled back to make the anchoring body 200 flip.

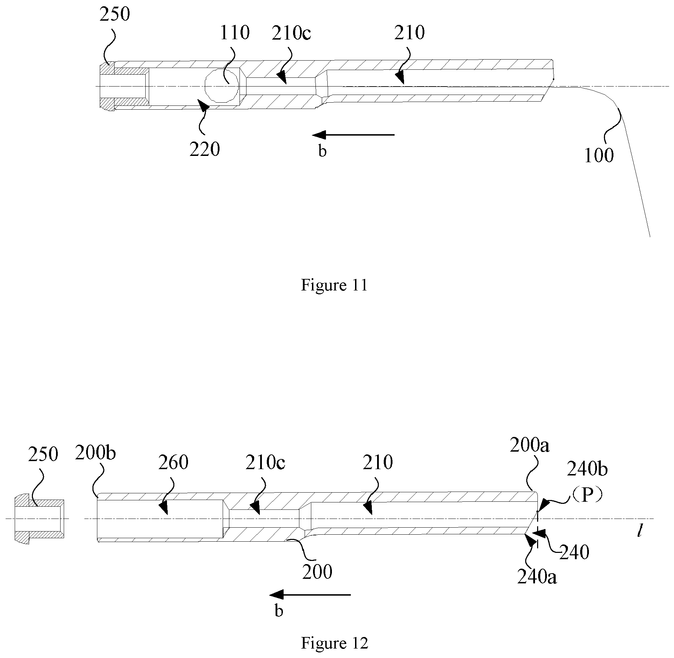

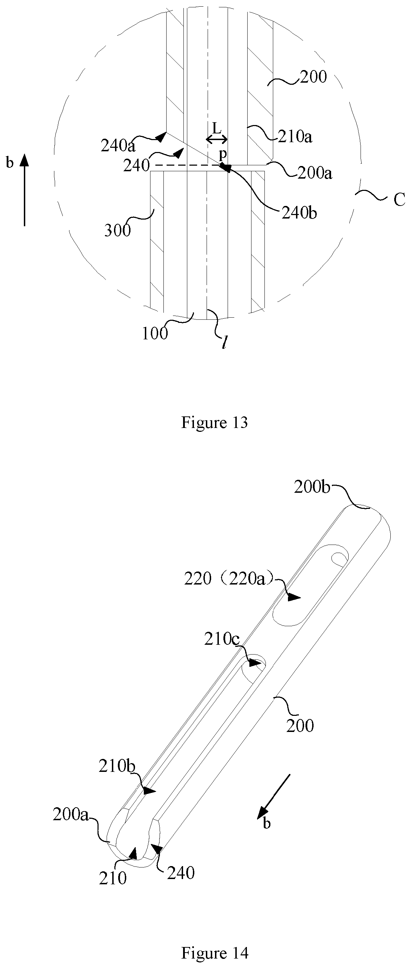

Figure 10 is a schematic diagram of the structure of one of the anchors provided in one embodiment of the present invention, Figure 11 is a sectional view of the assembly of the anchor and the traction line in Figure 10, Figure 12 is a sectional view of the anchor in Figure 11, and Figure 13 is a partial enlarged view of part C in Figure 6. Referring to Figures 10 to 13, in some examples, a limiting groove 210 and a side notch 210b communicating with the limiting groove 210 may be formed on the anchoring body 200. The limiting groove 210 and the side notch 210b both extend along the length direction of the anchoring body 200, and one ends of the limiting groove 210 and the side notch 210b both run through the proximal end of the anchoring body 200. The length direction of the anchoring body 200 may be shown in the direction b in Figures 10 to 13.

It can be understood that the side notch 210b may be an opening of the limiting groove 210 on the side wall of the anchoring body 200. For the convenience of description, the two opposite ends of the limiting groove 210 along the length direction can be respectively referred to as the first end and the second end, wherein the end that runs through the proximal end of the anchoring body 200 is referred to as the first end, and the end that faces away from the proximal end of the anchoring body 200 is referred to as the second end.

The first end of the limiting groove 210 runs through the proximal end of the anchoring body 200, indicating that the first end of the limiting groove 210 is provided with an end notch, which is communicating with the side notch 210b and has different orientations.

When the proximal end of the anchoring body 200 is located at the puncture needle 20, at least part of the traction line 100 is located in the limiting groove 210, and extends out of the anchoring body 200 from the end notch of the limiting groove 210 and extends into the driving tube 300. In the process of the traction line 100 pulling the anchoring body 200 to flip, a part of the traction line 100 is separated from the anchoring body 200 from the side notch 210b.

As an example, when the traction line 100 is fixed, it can be fixed at any position in the limiting groove 210, or it can be fixed at a position in the anchoring body 200 other than the limiting groove 210, as long as part of the traction line 100 is received in the limiting groove 210.

In the embodiment of the present invention, the limiting groove 210 is provided on the anchoring body 200 to limit the traction line 100 in the radial direction of the anchoring body 200, so that when the anchoring body 200 is in the puncture needle 20, the traction line 100 is located in the anchoring body 200, which plays a good receiving role for the traction line 100 and avoids the traction line 100 from rubbing against the puncture needle 20 and accumulating.

In some other examples, a mounting hole perpendicular to the length direction can be formed in the anchoring body 200, so that the distal end of the traction line 100 is fixed in the anchoring body 200 and extends to the outside of the anchoring body 200 through the mounting hole. When the proximal end of the anchoring body 200 is located at the puncture needle 20, the traction line 100 extends into the driving tube 300 along the outer wall of the anchoring body 200.

Referring to Figures 10 to 13, in order to achieve better flipping of the anchoring body 200, in some examples, the limiting groove 210 is located at one end (i.e., the first end) at the proximal end of the anchoring body 200 and is provided with a avoidance opening 240, one end of which extends to the side notch 210b, and the other end of which extends to the side of the axis *l* of the limiting groove 210 that is away from the side notch 210b. It should be noted that the axis of the limiting groove 210 is consistent with the axis of the anchoring body 200, that is, the limiting groove 210 is coaxially arranged with the anchoring body 200.

For the convenience of description, the portion of the limiting groove 210 having the avoidance opening 240 in the length direction may be referred to as the first portion, and the other portion of the limiting groove 210 in the length direction may be referred to as the second portion. The angle at which the groove wall of the second portion of the limiting groove 210 extends circumferentially around the axis *l* is the second angle, and the angle at which the groove wall of the first portion extends circumferentially around the axis *l* is the first angle.

Both the first angle and the second angle are less than 360°, so that a side notch 210b is formed on the side wall of the anchoring body 200, that is, the limiting groove 210 is not closed in the circumferential direction. The first angle is less than the second angle, so that the first end of the limiting groove 210 forms an avoidance opening 240.

In some examples, the first angle can be reduced in the direction from the second portion of the limiting groove 210 to the proximal end of the anchoring body 200, and the slope of the reduction is equal, so that the end surface of the avoidance opening 240 communicating with the side notch is formed as an inclined plane. Of course, in some other examples, when the slopes of the reduction of the first angle in the direction from the second portion of the limiting groove 210 to the proximal end of the anchoring body 200 are different, for example, the slopes of the reduction gradually increases, so that the end surface of the avoidance opening 240 communicating with the side notch is formed as an arc surface.

In some examples, the first angle is constant in the direction from the second portion of the limiting groove 210 to the proximal end of the anchoring body 200, then the end surface of the avoidance opening 240 communicating with the side notch is a vertical surface perpendicular to the side notch, and the avoidance opening 240 has a horizontal surface perpendicular to the vertical surface, that is, the entire end surface of the avoidance opening 240 is a right angle surface. The embodiment of the present invention does not limit the shape of the end surface of the avoidance opening 240.

In addition, the other end of the avoidance opening 240 extends to the side of the axis *l* of the limiting groove 210 that is away from the side notch 210b, that is, the first angle of the end of the first portion of the limiting groove 210 located at the proximal end of the anchoring body 200 is less than 180°, that is, the end groove wall of the first portion of the limiting groove 210 located at the proximal end of the anchoring body 200 extends circumferentially around the axis *l* to a plane below that where the axis *l* is located, that is, the other end of the avoidance opening 240 has a first distance from the axis *l* of the limiting groove 210 in the radial direction of the anchoring body 200 (refer to L in Figure 13). It can be understood that the plane where the axis *l* is located is arranged opposite to the groove bottom 210a.

By forming an avoidance opening 240 at the first end of the limiting groove 210, and one end of the avoidance opening 240 extends to the side notch 210b, and the other end extends to the side of the axis *l* of the limiting groove 210 away from the side notch 210b, so that the end surface of the proximal end of the anchoring body 200, except for the avoidance opening 240, abuts the distal end of the driving tube 300, and the end of the avoidance opening 240 away from the side notch 210b serves as the flip fulcrum P of the anchoring body 200.

When the proximal end of the anchoring body 200 reaches or is about to reach the distal end of the puncture needle 20, the traction line 100 is pulled, so that the flip fulcrum P is subjected to the pushing force of the distal end of the driving tube 300, and because the flip fulcrum P has a first distance from the axis *l* of the limiting groove 210, when the anchoring body 200 is pulled by the traction line 100, the flip fulcrum P is pushed by the distal end of the driving tube 300, thereby forming a flipping torque, so that the anchoring body 200 can achieve better flipping.

In some examples, there is a gap between the other end of the avoidance opening 240 (i.e., the flip fulcrum P) and the groove bottom 210a of the limiting groove 210. In other words, the other end of the avoidance opening 240 does not extend to the groove bottom 210a of the limiting groove 210, so that the proximal end of the anchoring body 200 reserves a portion of the end surface perpendicular to the axis *l*, so that the distal end of the driving tube 300 can stably abut against the end notch of the limiting groove 210 without entering the limiting groove 210, or shaking on the end notch of the limiting groove 210, so that the driving tube 300 can push the anchoring body 200 to move stably along the puncture needle 20, and the driving tube 300 can stably abut against the flip fulcrum P, and the anchoring body 200 can be pulled to flip stably under the pulling of the traction line 100.

The groove bottom 210a of the limiting groove 210 is arranged opposite to the side notch 210b.

As an example, the distance between the other end of the avoidance opening 240 and the groove bottom 210a of the limiting groove 210 can be less than or equal to 5 mm. For example, the distance can be a suitable value such as 1 mm, 3 mm or 5 mm. The embodiment of the present invention does not limit the distance.

In some examples, when the proximal end of the anchoring body 200 is located at the puncture needle 20, the traction line 100 can extend along the axis *l* of the limiting groove 210, so that the traction line 100 pulls the anchoring body 200 along the axis *l* of the limiting groove 210, so that the anchoring body 200 is flipped.

In other examples, when the proximal end of the anchoring body 200 is located at the puncture needle 20, the traction line 100 can be located between the axis *l* of the limiting groove 210 and the side notch 210b.

For the convenience of description, the side of the limiting groove 210 located on the axis *l* toward the side notch 210b can be referred to as the first side, and the side of the limiting groove 210 located on the axis *l* toward the flip fulcrum P (or the groove bottom 210a) can be referred to as the second side. The traction line 100 is arranged on the first side of the limiting groove 210 to deviate from the second side, that is, the flip fulcrum P. In this way, the traction line 100 pulls the anchoring body 200 along the side away from the flip fulcrum P, which can increase the flipping torque of the anchoring body 200, so that the anchoring body 200 can achieve better flipping under the pulling of the traction line 100 and the pushing of the driving tube 300.

In some examples, the distal end of the traction line 100 can be fixed at any position of the limiting groove 210, for example, it can be fixed inside the second end of a single limiting groove 210.

As shown in Figure 10, in some other examples, a wire placement cavity 220 may be formed on the anchoring body 200.

The line placement cavity 220 communicates with the groove cavity of the limiting groove 210 through the penetration hole 210c, the distal end of the traction line 100 is limited in the line placement cavity 220, and the traction line 100 extends from the penetration hole 210c into the limiting groove 210.

For example, the line placement cavity 220 and the limiting groove 210 may be arranged at intervals along the length direction of the anchoring body 200, so that the part of the traction line 100 located in the anchoring body 200 extends along the length direction of the anchoring body 200.

The penetration hole 210c is located in the anchoring body 200 and extends along the length direction of the anchoring body 200. The provision of the penetration hole 210c can better limit the specific position of the traction line 100 in the radial direction on the anchoring body 200, and simplify the matching structure of the anchoring body 200 and the traction line 100. For example, the penetration hole 210c can be arranged on the axis *l* of the limiting groove 210, that is, the axis *l* of the penetration hole 210c coincides with the axis *l* of the limiting groove 210. In this way, when the distal end of the traction line 100 is fixed in the line placement cavity 220 and the traction line 100 enters the limiting groove 210 along the penetration hole 210c, the traction line 100 can extend along the axis *l* of the limiting groove 210. Similarly, when the penetration hole 210c is arranged on the extension area of the first side of the limiting groove 210, the traction line 100 is limited to the extension area of the first side of the limiting groove 210.

In some examples, the distal end of the traction line 100 can be fixed in the line placement cavity 220 by bonding or welding.

In some other examples, the distal end of the traction line 100 is provided with a first limiting portion 110, and the first limiting portion 110 is snapped in the line placement cavity 220, so that the distal end of the traction line 100 is limited in the line placement cavity 220.

It can be understood that the width dimension of the first limiting portion 110 is greater than the opening width dimension of the line placement cavity 220, so that the first limiting portion 110 is clamped in the line placement cavity 220, and the distal end of the traction line 100 is limited in the line placement cavity 220 and will not detach from the notch of the line placement cavity 220. In addition, the outer contour dimension of the first limiting portion 110 is greater than the first orifice of the penetration hole 210c, so that the first limiting portion 110 will not enter the limiting groove 210 from the penetration hole 210c, ensuring that the first limiting portion 110, i.e. the distal end of the traction line 100, is limited in the line placement cavity 220.

In some examples, the first limiting portion 110 can be movable in the line placement cavity 220 or fixed in the line placement cavity 220, and the embodiments of the present invention are not limited to this.

For example, the first limiting portion 110 can be an integral part with the distal end of the traction line 100, for example, the first limiting portion 110 can be a knot at the distal end of the traction line 100. In some examples, the first limiting portion 110 can also be a block or spherical limit structure fixed at the distal end of the traction line 100.

In some examples, when the anchoring body 200 flips to the anchoring position, the position where the traction line 100 is detached from the anchoring body 200 is located at the center position of the anchoring body 200 along the length direction.

It can be understood that when the anchoring body 200 flips to the anchoring position, a part of the traction line 100 is located inside the anchoring body 200, and the other part is separated from the anchoring body 200, that is, the other part is located outside the anchoring body 200, then the position where the traction line 100 is detached from the anchoring body 200 (as shown in M in Figure 7) refers to the position on the anchoring body 200 where the turning point of the part of the traction line 100 located inside the anchoring body 200 and the part of the traction line 100 located outside the anchoring body 200 corresponds to.

By arranging the position where the traction line 100 is detached from the anchoring body 200 at the center position of the anchoring body 200 along the length direction, the anchoring body 200 will not deviate from the anchoring position during the pulling process of the traction line 100, that is, the angle between the anchoring body 200 and the traction line 100 will not deviate from the anchoring angle, ensuring that the anchoring body 200 can stably fit with the inner wall of the target tissue 40 during the pulling process of the traction line 100 without causing damage to the target tissue 40.

It is understandable that when the anchoring body 200 is flipped to the anchoring position, the position where the traction line 100 is detached from the anchoring body 200 is the second end of the limiting groove 210. Therefore, the second end of the limiting groove 210 can be extended to the center position of the anchoring body 200, so that the position where the traction line 100 is detached from the anchoring body 200 is the center position of the anchoring body 200.

In some examples, the distal end of the traction line 100 can be directly fixed to the center position of the anchoring body 200, for example, it can be fixed to the second end of the limiting groove 210.

In other examples, the distal end of the traction line 100 can be located between the center position of the anchoring body 200 and the distal end of the anchoring body 200. For example, the distal end of the traction line 100 is limited in the above-mentioned line placement cavity 220. Since the above-mentioned line placement cavity 220 is located on the side of the limiting groove 210 toward the distal end of the anchoring body 200, for example, the line placement cavity 220 is located between the center position of the anchoring body 200 and the distal end of the anchoring body 200, the distal end of the traction line 100 deviates from the center position of the anchoring body 200, for example, it can be located between the center position of the anchoring body 200 and the distal end of the anchoring body 200, and the traction line 100 extends toward the proximal end of the anchoring body 200.

Referring to Figures 11 to 12, in some examples, a mounting channel 260 is formed on the anchoring body 200, one end of the mounting channel 260 communicates with the penetration hole 210c, and the other end of the mounting channel 260 runs through the distal end of the anchoring body 200. For example, the mounting channel 260 is located on the side of the limiting groove 210 toward the distal end of the anchoring body 200, and the penetration hole 210c is located between the mounting channel 260 and the limiting groove 210, and communicates the mounting channel 260 with the limiting groove 210.

In some examples, the distal end of the anchoring body 200 has an end cap 250, which is embedded in the mounting channel 260 from the distal end of the anchoring body 200, and one end of the end cap 250 is spaced apart from the penetration hole 210c, and a line placement cavity 220 is formed between one end of the end cap 250 and the penetration hole 210c.

For example, the penetration hole 210c has a first orifice and a second orifice that are arranged oppositely along the length direction of the anchoring body 200, wherein the first orifice faces the limiting groove 210 and communicates with the limiting groove 210, and the end surface where the second orifice is located is spaced apart from one end of the end cap 250, and the end surface where the second orifice is located and one end of the end cap 250 form a line placement cavity 220.

By providing a mounting channel 260 on the anchoring body 200, making the mounting channel 260 extend to the distal end of the anchoring body 200, and providing an end cap 250 at the distal end of the anchoring body 200 to form a line placement cavity 220, the structural arrangement of the anchoring body 200 is simplified, and the distal end of the traction line 100 is conveniently assembled in the line placement cavity 220.

Figure 14 is a schematic diagram of the structure of another anchor provided by an embodiment of the present invention. Referring to Figure 14, in some other examples, the side wall of the anchoring body 200 is recessed inward to form a line placement groove 220a, and the groove cavity of the line placement groove 220a is configured as a line placement cavity 220. The above specific embodiments further explain in detail the purpose, technical solution and beneficial effects of the embodiments of the present invention. It should be understood that the above embodiment is only a specific embodiments of the embodiments of the present invention, and is not used to limit the protection scope of the embodiments of the present invention. Any modifications, equivalent replacements, improvements, etc. made on the basis of the technical solution of the embodiments of the present invention should be included in the protection scope of the embodiments of the present invention.

## Claims

1. An anchor (10), **characterized by** comprising:
a traction line (100), wherein the traction line (100) is a flexible member;
an anchoring body (200), connected to a distal end of the traction line (100) and capable of being pulled by the traction line (100); and
a driving tube (300), at least sleeved on at least a portion of the traction line (100), wherein the anchoring body (200) is configured to move along a puncture needle (20) under drive of the driving tube (300), and can be flipped when extending out of a distal end of the puncture needle (20) to form an angle with the traction line (100), so that the anchoring body (200) stops at an inner wall of a target tissue.

2. The anchor (10) according to claim 1, wherein the driving tube (300) is sleeved on the traction line (100), and the anchoring body (200) is located at a distal end of the driving tube (300); and
when the anchoring body (200) is located in the puncture needle (20), a proximal end of the anchoring body (200) abuts the distal end of the driving tube (300), so that when the anchoring body (200) extends out of the puncture needle (20), the anchoring body (200) can be flipped under pushing of the driving tube (300) and pulling of the traction line (100).

3. The anchor (10) according to claim 2, wherein the anchor (10) further comprises:
a distal locking structure (400), configured to be fixed to a proximal end of the puncture needle (20), wherein a first receiving channel (430) is formed in the distal locking structure (400), the first receiving channel (430) communicates with a needle channel of the puncture needle (20), a part of the driving tube (300) and the anchoring body (200) are located in the first receiving channel (430), and the anchoring body (200) is configured to enter the needle channel of the puncture needle (20) through the first receiving channel (430) under the drive of the driving tube (300); and
the distal locking structure (400) is configured to selectively cooperate with the driving tube (300) to lock the driving tube (300) and the anchoring body (200) in the first receiving channel (430).

4. The anchor (10) according to claim 3, wherein the distal locking structure (400) comprises:
a distal connecting member (410), configured to be fixed to the proximal end of the puncture needle (20) and to form at least a portion of the first receiving channel (430) for the driving tube (300) and the anchoring body (200) to pass through; and
a distal locking member (420), disposed on the distal connecting member (410) and configured to selectively cooperate with the driving tube (300) to lock or release the driving tube (300) and the anchoring body (200) on the distal connecting member (410).

5. The anchor (10) according to claim 4, wherein the distal locking member (420) comprises:
a distal fixing portion (421), wherein the distal fixing portion (421) is provided with the first receiving channel (430), and is selectively fixed to or released from the distal connecting member (410); and
a distal locking portion (422), wherein the distal locking portion (422) is located inside the distal fixing portion (421),
wherein when the distal fixing portion (421) is fixed to the distal connecting member (410), the distal locking portion (422) is configured to cooperate with the distal connecting member (410) and act on the driving tube (300) to lock the driving tube (300) and the anchoring body (200); and
wherein when the distal fixing portion (421) is released from the distal connecting member (410), the distal locking portion (422) is configured to detach from the distal connecting member (410) to release the driving tube (300) and the anchoring body (200).

6. The anchor (10) according to claim 5, wherein the distal locking portion (422) is a distal elastic member embedded in the distal fixing portion (421), and the distal elastic member is elastic at least along a first direction; the first direction intersects with an extension direction of the driving tube (300),
wherein when the distal fixing portion (421) is fixed to the distal connecting member (410), the distal elastic member is pressed by the distal connecting member (410) and abuts the driving tube (300) along the first direction to lock the driving tube (300) and the anchoring body (200); and
wherein when the distal fixing portion (421) is released from the distal connecting member (410), the distal elastic member detaches from the distal connecting member (410) to release the driving tube (300) and the anchoring body (200) during a rebound process,
preferably, the distal elastic member is a distal elastic sleeve;
the distal elastic sleeve is provided with a first penetration hole for the driving tube (300) to pass through; and
when the distal elastic sleeve is pressed by the distal connecting member (410), a hole wall of the first penetration hole abuts the driving tube (300) to lock the driving tube (300) and the anchoring body (200), and after the distal elastic sleeve is detached from the distal connecting member (410), the hole wall of the first penetration hole rebounds to release the driving tube (300) and the anchoring body (200);
preferably, the distal fixing portion (421) is a distal locking nut, and the distal locking nut is threadedly connected to the distal connecting member (410).

7. The anchor (10) according to claim 1, wherein the anchor (10) further comprises:
a proximal locking structure (500), wherein the proximal locking structure (500) is arranged at a proximal end of the driving tube (300) and is fixedly connected to the driving tube (300);
a second receiving channel (530) is formed in the proximal locking structure (500), and the traction line (100) is passed through the second receiving channel (530); and
the proximal locking structure (500) is configured to selectively cooperate with the traction line (100) to lock the traction line (100) on the driving tube (300).

8. The anchor (10) according to claim 7, wherein the proximal locking structure (500) comprises:
a proximal connecting member (510), fixed to the proximal end of the driving tube (300), and forming at least a portion of the second receiving channel (530) for the traction line (100) to pass through; and
a proximal locking member (520), arranged on the proximal connecting member (510), and configured to selectively cooperate with the traction line (100) to lock or release the traction line (100) on the proximal connecting member (510),
preferably, the proximal locking member (520) comprises:
a proximal fixing portion (521), wherein the proximal fixing portion (521) is provided with the second receiving channel (530) and selectively fixed to or released from the proximal connecting member (510); and
a proximal locking portion (522), wherein the proximal locking portion (522) is located inside the proximal fixing portion (521),
wherein when the proximal fixing portion (521) is fixed to the proximal connecting member (510), the proximal locking portion (522) is configured to cooperate with the proximal connecting member (510) and act on the traction line (100) to lock the traction line (100); and
wherein when the proximal fixing portion (521) is detached from the proximal connecting member (510), the proximal locking portion (522) is configured to detach from the proximal connecting member (510) to release the traction line (100).

9. The anchor (10) according to claim 8, wherein the proximal locking portion (522) is a proximal elastic member embedded in the proximal fixing portion (521), and the proximal elastic member is elastic at least along a first direction; the first direction intersects with an extension direction of the traction line (100),
wherein when the proximal fixing portion (521) is fixed to the proximal connecting member (510), the proximal elastic member is pressed by the proximal connecting member (510) and abuts the traction line (100) along the first direction to lock the traction line (100); and
wherein when the proximal fixing portion (521) is released from the proximal connecting member (510), the proximal elastic member detaches from the proximal connecting member (510) to release the traction line (100) during a rebound process,
preferably, the proximal elastic member is a proximal elastic sleeve;
the proximal elastic sleeve is provided with a second penetration hole for the traction line (100) to pass through; and
when the proximal elastic sleeve is pressed by the proximal connecting member (510), a hole wall of the second penetration hole abuts the traction line (100) to lock the driving tube (300), and after the proximal elastic sleeve is detached from the proximal connecting member (510), the hole wall of the penetration hole rebounds to release the traction line (100);
preferably, the proximal fixing portion (521) is a proximal locking nut, and the proximal locking nut is threadedly connected to the proximal connecting member (510).

10. The anchor (10) according to any one of claims 1 to 9, wherein the anchor (10) further comprises:
a stopper (600), selectively arranged between the driving tube (300) and the puncture needle (20),
wherein when the anchoring body (200) is located in the puncture needle (20) and has a preset distance from the distal end of the puncture needle (20), the stopper (600) cooperates with the driving tube (300) and the puncture needle (20) respectively to limit a movement of the driving tube (300) in the puncture needle (20); and
wherein during a movement of the anchoring body (200) from a first position to the distal end of the puncture needle (20), the stopper (600) is detached from a position between the driving tube (300) and the puncture needle (20), wherein the first position is a position of the anchoring body (200) when having the preset distance from the distal end of the puncture needle (20).

11. The anchor (10) according to claim 10, wherein the stopper (600) is arranged between a proximal locking structure (500) and a distal locking structure (400) of the anchor (10) and selectively protrudes from a side wall of the driving tube (300);
at least when the anchoring body (200) is located in the puncture needle (20) and has the preset distance from the distal end of the puncture needle (20), the stopper (600) protrudes from the side wall of the driving tube (300) so that a proximal end of the driving tube (300) is blocked by the stopper (600) on a proximal end of the puncture needle (20); and
during the movement of the anchoring body (200) from the first position to the distal end of the puncture needle (20), the stopper (600) is detached from the side wall of the driving tube (300),
preferably, the stopper (600) is detachably arranged on the driving tube (300); and
when the anchoring body (200) moves from the first position toward the distal end of the puncture needle (20), the stopper (600) is configured to be detached from the driving tube (300);
preferably, the stopper (600) is arranged at one end of the proximal locking structure (500) of the anchor (10) toward the distal locking structure (400); and
the preset distance is 0, and a length of the stopper (600) is equal to a maximum length of the anchoring body (200) extending out of the distal end of the puncture needle (20).

12. The anchor (10) according to any one of claims 1 to 9, wherein a limiting groove (210) and a side notch (210b) communicated with the limiting groove (210) are formed on the anchoring body (200), the limiting groove (210) and the side notch (210b) both extend along a length direction of the anchoring body (200), and one ends of the limiting groove (210) and the side notch (210b) both run through a proximal end of the anchoring body (200),
wherein when the proximal end of the anchoring body (200) is located at the puncture needle (20), at least part of the traction line (100) is located in the limiting groove (210), and during a process of the traction line (100) pulling the anchoring body (200) to flip, a part of the traction line (100) detaches from the anchoring body (200) through the side notch (210b).

13. The anchor (10) according to claim 12, wherein an avoidance opening (240) is formed at one end of the limiting groove (210) located at the proximal end of the anchoring body (200), one end of the avoidance opening (240) extends to the side notch (210b), and the other end of the avoidance opening (240) extends to a side of an axis of the limiting groove (210) away from the side notch (210b),
preferably, a gap is provided between the other end of the avoidance opening (240) and a groove bottom (210a) of the limiting groove (210); and
the groove bottom (210a) of the limiting groove (210) is arranged opposite to the side notch (210b);
preferably, when the proximal end of the anchoring body (200) is located at the puncture needle (20), the traction line (100) extends along the axis of the limiting groove (210); or
when the proximal end of the anchoring body (200) is located at the puncture needle (20), the traction line (100) is located between the axis of the limiting groove (210) and the side notch (210b).

14. The anchor (10) according to claim 12, wherein a line placement cavity (220) is formed on the anchoring body (200),
wherein the line placement cavity (220) communicates with a groove cavity of the limiting groove (210) through a penetration hole (210c), a distal end of the traction line (100) is limited in the line placement cavity (220), and the traction line (100) extends from the penetration hole (210c) into the limiting groove (210),
preferably, a first limiting portion (110) is formed at the distal end of the traction line (100), and the first limiting portion (110) is embedded in the line placement cavity so that the distal end of the traction line (100) is limited in the line placement cavity;
preferably, a side wall of the anchoring body (200) is recessed inwardly to form a line placement groove (220a), and a groove cavity of the line placement groove (220a) is configured as the line placement cavity;
preferably, a mounting channel (260) is formed in the anchoring body (200), one end of the mounting channel (260) communicates with the penetration hole (210c), and the other end of the mounting channel (260) penetrates to a distal end of the anchoring body (200),
wherein the distal end of the anchoring body (200) has an end cap (250), the end cap (250) is embedded in the mounting channel (260) from the distal end of the anchoring body (200), and one end is spaced from the penetration hole (210c); and
the line placement cavity is formed between one end of the end cap (250) and the penetration hole (210c).

15. An anchoring device, **characterized by** comprising:
a puncture needle (20), wherein a distal end of the puncture needle (20) is configured to extend into a target tissue; and
the anchor (10) according to any one of claims 1 to 14,
wherein the anchoring body (200) of the anchor (10) moves along the puncture needle (20) under the drive of the driving tube (300) to enter the target tissue.
